(19) Europäisches Patentamt
European Patent Office
Office européen des brevets

(11) EP 4 745 118 A1

(12) **EUROPEAN PATENT APPLICATION**
published in accordance with Art. 153(4) EPC

(43) Date of publication:
**20.05.2026 Bulletin 2026/21**

(21) Application number: **24850699.0**

(22) Date of filing: **26.06.2024**

(51) International Patent Classification (IPC):
***C07C 259/06** (2006.01)* ***A61K 31/166** (2006.01)*
***A61P 31/04** (2006.01)*

(52) Cooperative Patent Classification (CPC):
**C07C 259/06; A61P 31/04;** C07B 2200/07; C07C 2601/02; Y02A 50/30

(86) International application number:
**PCT/CN2024/101528**

(87) International publication number:
**WO 2025/031048 (13.02.2025 Gazette 2025/07)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **08.08.2023 CN 202310989119**

(71) Applicant: **Institute of Medicinal Biotechnology, Chinese Academy of Medical Sciences**
**Beijing 100050 (CN)**

(72) Inventors:
- **WANG, Yucheng**
  **Beijing 100050 (CN)**
- **YOU, Xuefu**
  **Beijing 100050 (CN)**
- **WANG, Minghua**
  **Beijing 100050 (CN)**
- **ZHANG, Guoning**
  **Beijing 100050 (CN)**
- **DU, Xiaonan**
  **Beijing 100050 (CN)**
- **WANG, Juxian**
  **Beijing 100050 (CN)**
- **HU, Xinxin**
  **Beijing 100050 (CN)**
- **WANG, Xiukun**
  **Beijing 100050 (CN)**
- **LI, Congran**
  **Beijing 100050 (CN)**
- **NIE, Tongying**
  **Beijing 100050 (CN)**
- **XIE, Chunyang**
  **Beijing 100050 (CN)**

(74) Representative: **karo IP**
**Patentanwälte PartG mbB**
**Steinstraße 16-18**
**40212 Düsseldorf (DE)**

(54) **HYDROXAMIC ACID COMPOUND OR PHARMACEUTICALLY ACCEPTABLE SALT THEREOF, AND USE THEREOF AND PREPARATION METHOD THEREFOR**

(57) This application relates to a compound as shown in the following formula (1) or a pharmaceutically acceptable salt thereof, wherein R is $C_{1-6}$ alkyl. This application also relates to the use of the compound or the pharmaceutically acceptable salt thereof for preparing a drug against Gram-negative bacteria, and a method for preparing the compound or the pharmaceutically acceptable salt thereof.

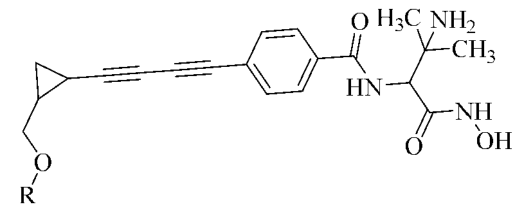

(1)

EP 4 745 118 A1

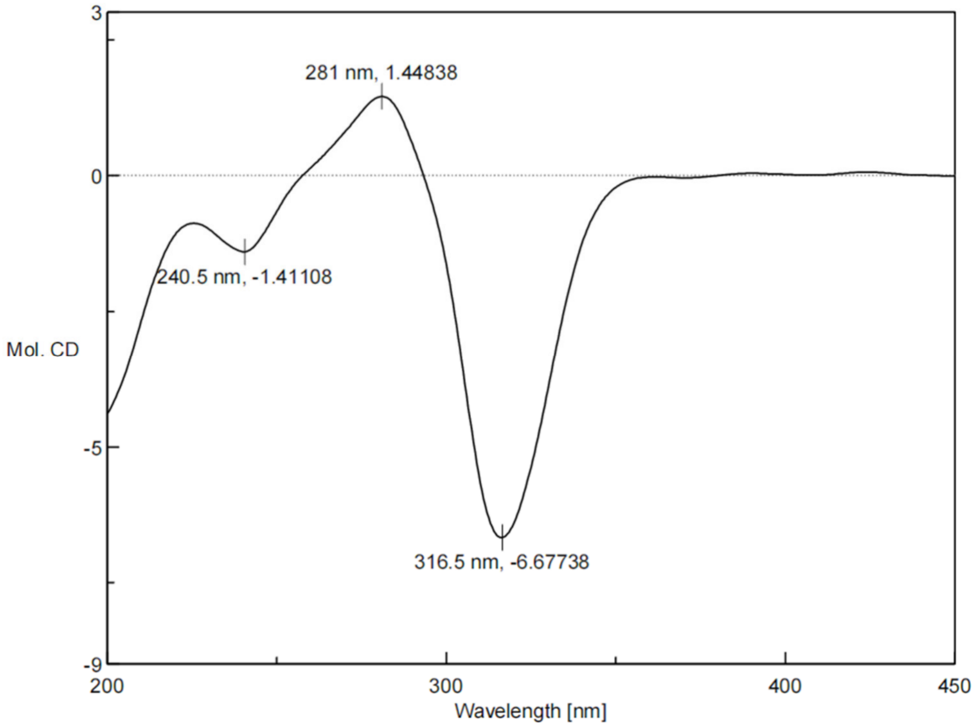
3
0
-5
-9
Mol. CD
281 nm, 1.44838
240.5 nm, -1.41108
316.5 nm, -6.67738
200
300
400
450
Wavelength [nm]

Fig. 1

## Description

### Technical Field

This application relates to a novel hydroxamic acid compound or a pharmaceutically acceptable salt thereof, the use of the compound or the pharmaceutically acceptable salt thereof for preparing a drug against Gram-negative bacteria, and a method for preparing the compound or the pharmaceutically acceptable salt thereof.

### Background Art

Bacterial resistance is becoming increasingly severe, especially in Gram-negative bacteria, where the number of deaths from bacterial infections is increasing year by year, posing a serious threat to human life and health. Although many antibacterial drugs have been developed, such as β - lactams, aminoglycosides, fluoroquinolones, glycopeptides, etc., they all have varying degrees of resistance or toxicity, which greatly limits their application.

UDP-3-O (R-hydroxytetradecanoyl)-N-acetylglucosamine deacetylase (LpxC) is a key enzyme that catalyzes the synthesis of lipid A, the main component of the outer membrane lipopolysaccharide in Gram-negative bacteria. The absence or overexpression of LpxC can cause the death of certain Gram-negative bacteria, making it an ideal target for drugs against Gram-negative bacteria. Although there have been many advances in this field, there is still a need for new LpxC inhibitors that not only have bactericidal activity against Gram-negative bacteria, but also have acceptable toxicity or tolerability.

Prior art such as Chinese Patent Application CN10329878A discloses the LpxC inhibitor ACHN-975 with the following structure. This compound, developed by Achaogen, was among the LpxC inhibitors that had progressed to Phase I clinical trials. However, due to certain reasons, it ultimately failed to become a drug.

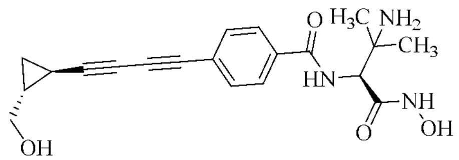

ACHN-975

### Summary of Invention

The present invention aims to provide a novel LpxC inhibitor with better activity and lower toxicity.

Specifically, the present invention provides, on the one hand, a compound as shown in the following formula (1) or a pharmaceutically acceptable salt thereof,

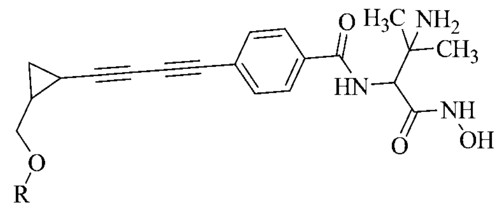

(1).

wherein R is alkyl, cycloalkyl, aralkyl, preferably lower alkyl, and more preferably $C_{1-6}$ alkyl.

The present invention provides, on the other hand, a compound as shown in the following formula (2) or a pharmaceutically acceptable salt thereof,

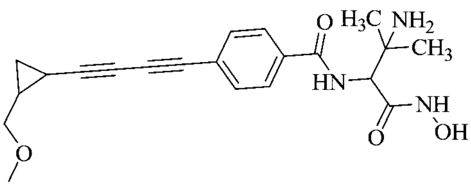

(2).

The present invention provides, in a further aspect, a compound as shown in the following formula (I) or a pharmaceutically acceptable salt thereof,

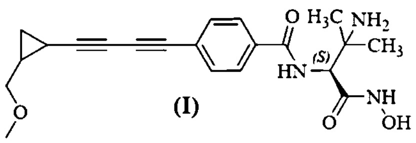
(I)

The present invention provides, in a further aspect, compounds as shown in any one of the following formula (Ia)-(Id), or their pharmaceutically acceptable salts,

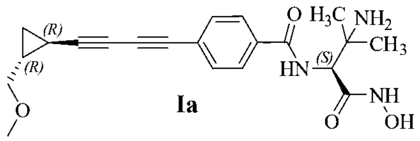
Ia

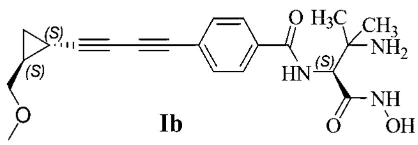
Ib

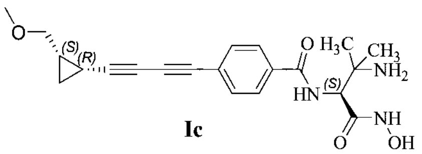
Ic

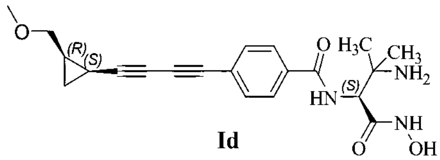
Id

The present invention further relates to a pharmaceutical composition comprising the compound of the above formula (1) or the pharmaceutically acceptable salt thereof and a pharmaceutically acceptable carrier. The compound of formula (1) is preferably the compound of formula (2), more preferably the compound of formula (I), further preferably one or more compounds selected from the group consisting of (Ia), (Ib), (Ic), and (Id), and particularly preferably the compound of formula (Ia).

The present invention further relates to the use of the above compounds or their pharmaceutically acceptable salts for preparing a drug against Gram-negative bacteria. The Gram-negative bacteria include, but are not limited to, Escherichia coli, Klebsiella pneumoniae, Pseudomonas aeruginosa, Enterobacter cloacae, Enterobacter aerogenes, Serratia marcescens, Citrobacter freundii, Pantoea agglomerans, Proteus vulgaris, Proteus mirabilis and Shigella flexneri, etc..

The present invention further relates to a method for preparing the compound as shown in the above formula Ia, which comprises any one or more of the following steps:

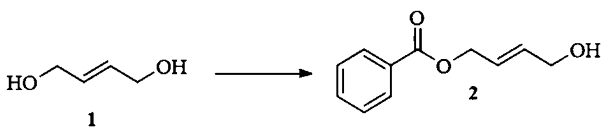
1
2

Step 1

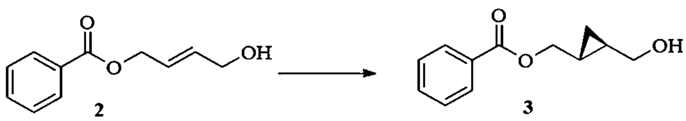
2
3

Step 2

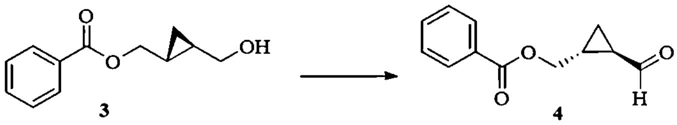
3
4

Step 3

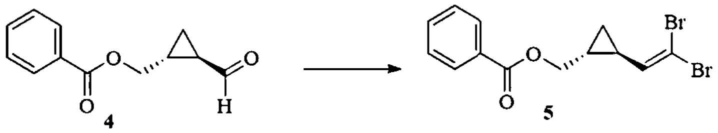

4 5

Step 4

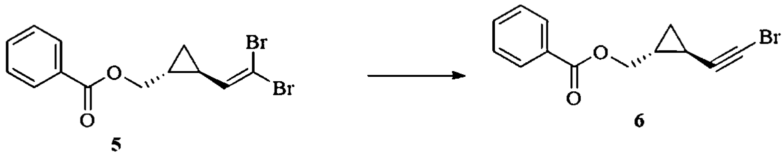

5 6

Step 5

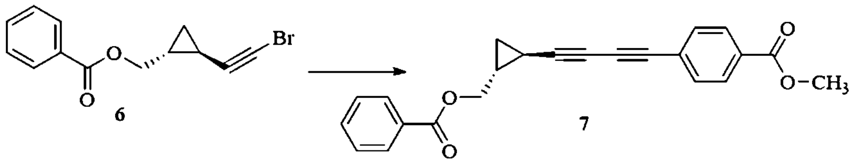

6 7

Step 6

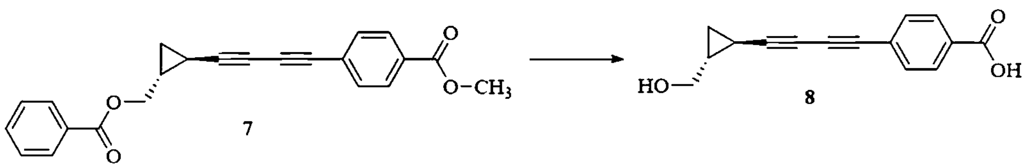

7 8

Step 7

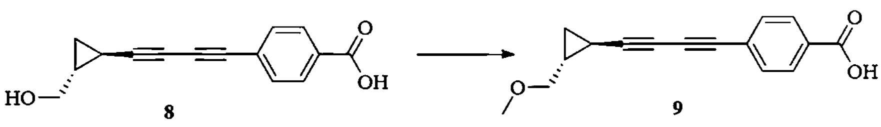

8 9

Step 8

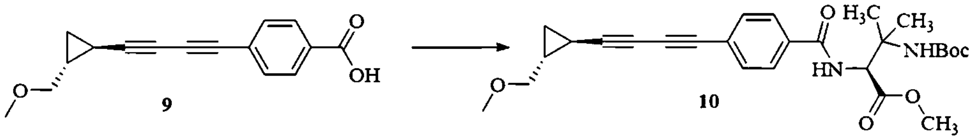

9 10

Step 9

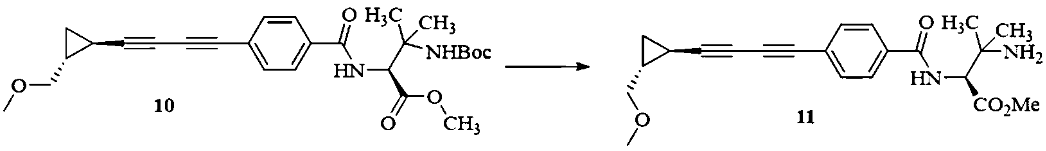

10 11

Step 10

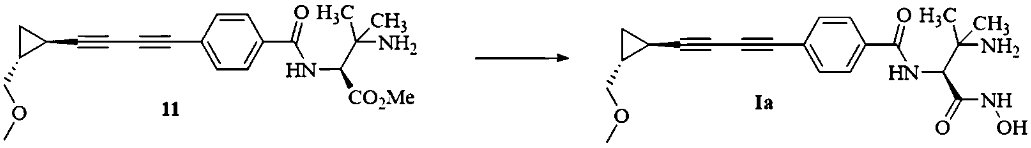

11 Ia

Step 11.

The present invention further relates to a method for preparing the compounds as shown in the above formula Ib, Ic or Id, which comprises any one or more of the following steps, wherein Ib uses *trans*-2-butene-1,4-diol as the starting material, and formula Ic and Id use *cis*-2-butene-1,4-diol as the starting material, with the cross-link in the formula denoting either *cis* or *trans* configuration.

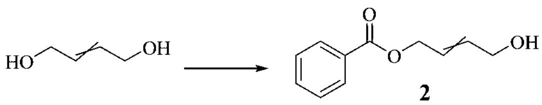

2

Step 1'

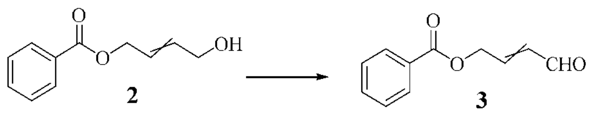

2 3

Step 2'

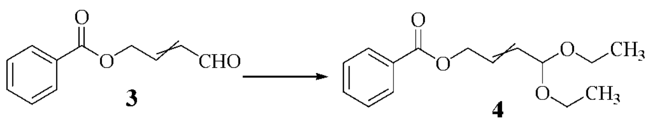

3 4

Step 3'

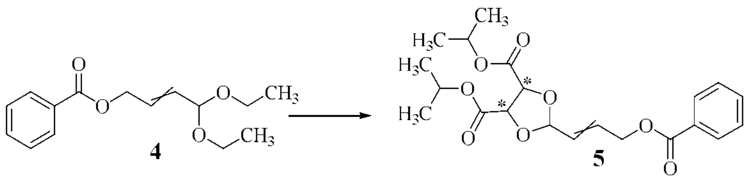

Step 4'

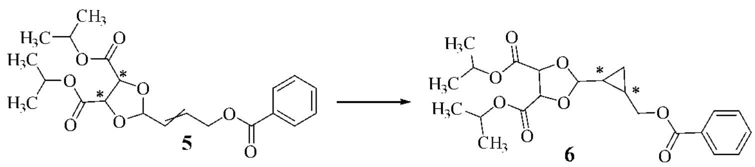

Step 5'

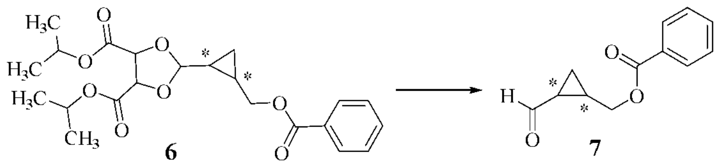

Step 6'

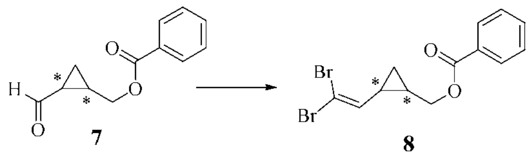

Step 7'

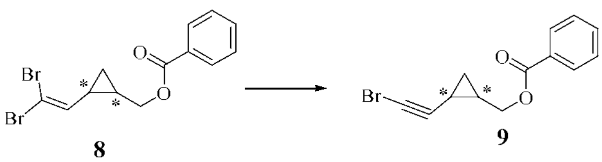

Step 8'

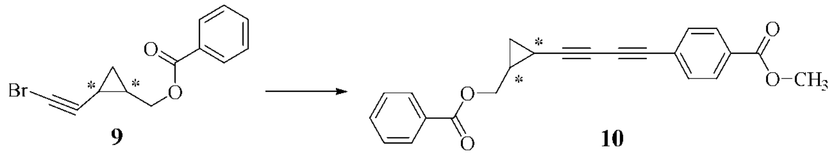

9 10

Step 9'

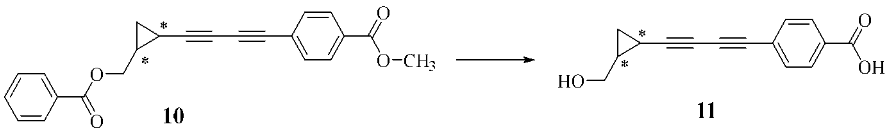

10 11

Step 10'

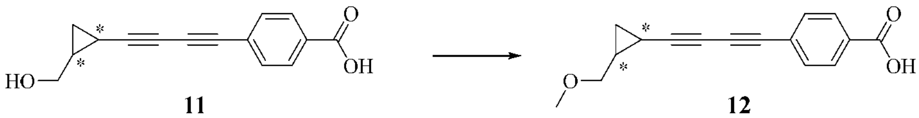

11 12

Step 11'

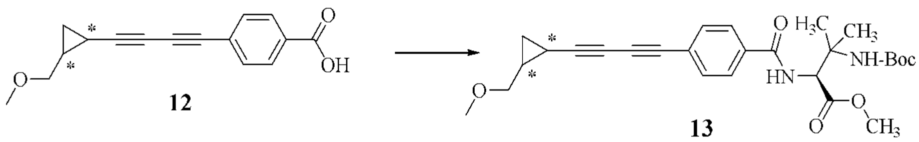

12 13

Step 12'

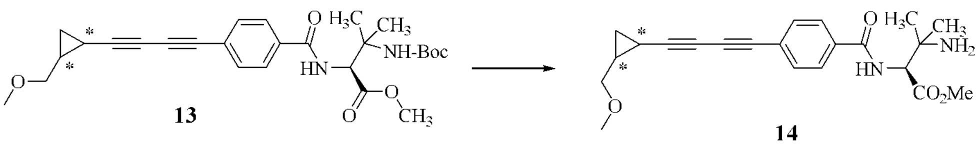

13 14

Step 13'

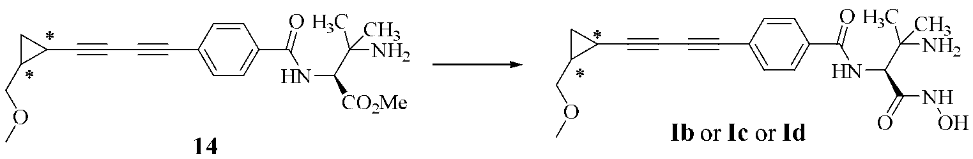

14 **Ib** or **Ic** or **Id**

Step 14'.

**Description of Drawings**

Figure 1 shows the circular dichroism spectrum of Compound 8 in Example 1.
Figure 2 shows the single crystal X-ray diffraction pattern of Compound 8 in Example 1.
Figure 3 shows the circular dichroism spectrum of Compound 11 in Example 4.

**Specific Embodiments**

In the compounds as shown in formula (1) as described above in this application or their pharmaceutically acceptable salts, R is alkyl, cycloalkyl, arylalkyl, preferably lower alkyl, more preferably $C_{1-6}$ alkyl. Specific examples include, but are not limited to, methyl, ethyl, n-propyl, isopropyl, n-butyl, sec-butyl, tert-butyl, etc.. Particularly preferably, R is methyl.

Preferably, the compound of formula (1) is the compound of formula (2) as described above in this application.

More preferably, the compound of (2) is the compound of (I) as described above in this application.

The compounds of Formula (1), (2), and (I) in this application can be prepared by methods described in known literatures such as CN101765585A and CN103298780A.

In this application, the term "pharmaceutically acceptable salt" denotes a salt formed by the compound of the present invention with an acid, wherein the acid may be selected from, but is not limited to, trifluoroacetic acid, phosphoric acid, sulfuric acid, hydrochloric acid, hydrobromic acid, citric acid, maleic acid, succinic acid, fumaric acid, acetic acid, lactic acid, p-toluenesulfonic acid, malic acid, and methanesulfonic acid.

In the compounds of formula (1), (2), and (I) of this application, the ternary ring contains two chiral carbon atoms and contains four isomers, making their preparation, separation, and purification extremely difficult. The present invention is the first to prepare and isolate isomeric compounds of Ia, Ib, Ic, and Id as described above.

The present invention uses 2-butene-1,4-diol as the starting material to prepare the corresponding optical isomers through de novo construction of the ternary ring chiral center. Formula Ia and Ib start from *trans*-2-butene-1,4-diol, while formula Ic and Id start from *cis*-2-butene-1,4-diol.

In Formula Ia, a chiral ternary ring is constructed through a one-step asymmetric Simmons-Smith cyclopropanation reaction. In formula Ib, Ic, and Id, a chiral center of a ternary ring is constructed through a separating four-step reaction with a chiral auxiliary reagent of diisopropyl D/L-(±)-tartrate.

Specifically, the present application provides a method for preparing the compound of Ia, which comprises:

(1) subjecting *trans*-2-butene-1,4-diol to an esterification reaction with benzoyl chloride under basic conditions to provide benzoyl-mono-protected 2-butene-1,4-diol;

(2) subjecting benzoyl-mono-protected 2-butene-1,4-diol to an asymmetric Simmons-Smith cyclopropanation reaction using an asymmetric inducer to provide ((1R,2R)-2-(hydroxymethyl)cyclopropyl)methyl benzoate;

(3) subjecting ((1R,2R)-2-(hydroxymethyl)cyclopropyl)methyl benzoate to an oxidation with Dess-Martin periodinane, Corey-Fuchs reaction, debromination, Cadiot-Chodkiewicz reaction, hydrolysis, methylation, condensation with methyl (S)-2-amino-3-(tert-butoxycarbonylamino)-3-methylbutanoate, deprotection, and hydroxylaminolysis to provide the optical isomer Ia.

In the asymmetric Simmons-Smith cyclopropanation reaction, the asymmetric inducer is preferably a chiral butyl borate or chiral sulphonamide ligand, more preferably 2-butyl-N,N,N',N'-tetramethyl-dioxaborolane-(4R,5R)-dicarboxamide; the amount of the asymmetric inducer relative to the starting compound is 1.05 to 1.1 equivalents.

In the oxidation reaction with the Dess-Martin periodinane, the molar ratio of the starting compound to the Dess-Martin periodinane is preferably 1: (1.0-1.2), the temperature is preferably room temperature, and the oxidation reaction time is preferably 2-5 hours.

In the Corey-Fuchs reaction, the reaction temperature is preferably -20 to -78°C.

More specifically, the present application provides a method for preparing the compound of Ia, which comprises any one or more of the following steps:

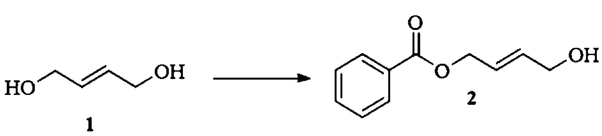

Step 1

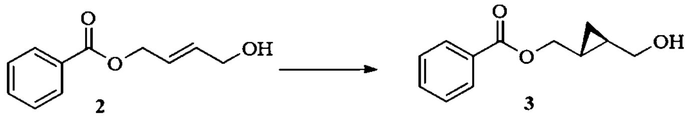

Step 2

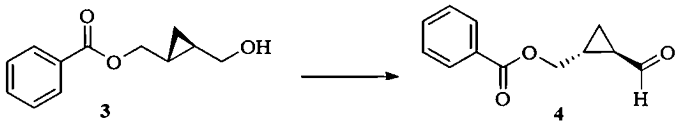

Step 3

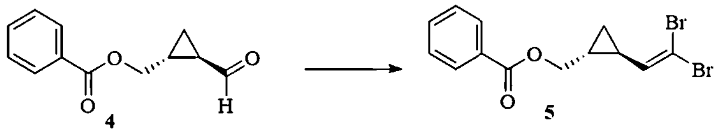

Step 4

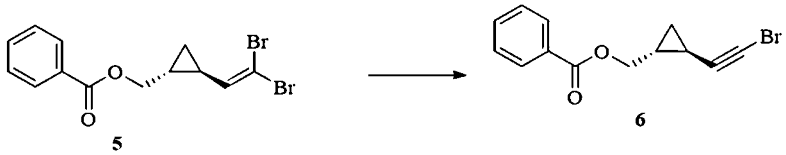

Step 5

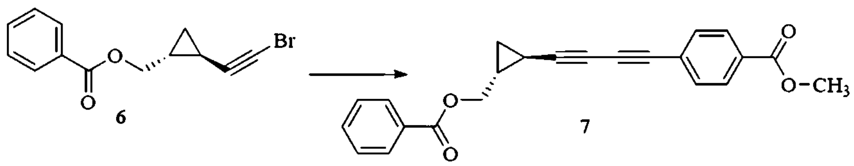

Step 6

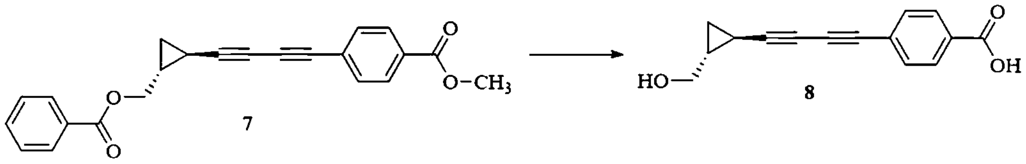

Step 7

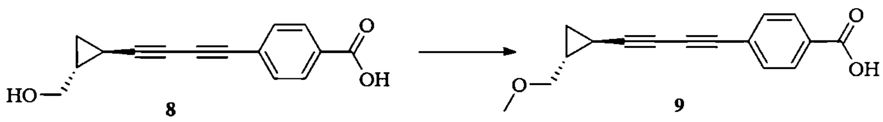

Step 8

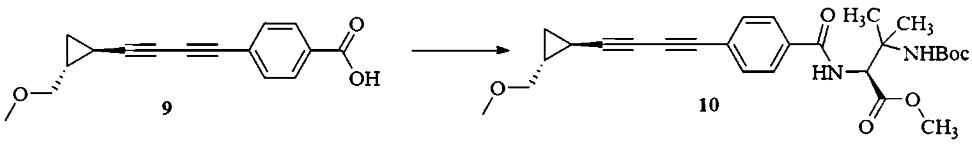

Step 9

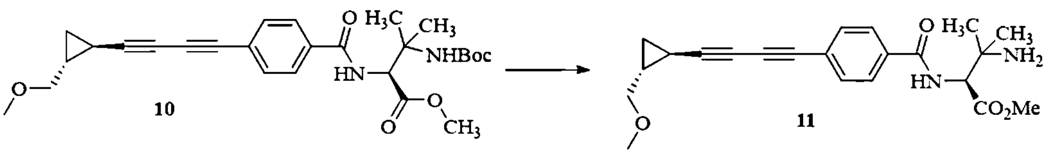

Step 10

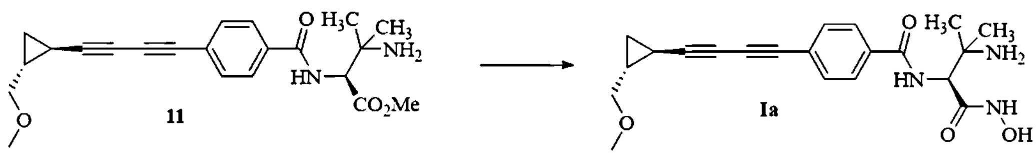

Step 11.

Step 1 can be performed as follows, but is not limited thereto. Dissolve Compound 1 in an organic solvent, add a base at -5°C to -10°C, slowly add benzoyl chloride dropwise, and stir at the same temperature. After the reaction is complete, isolate and obtain Compound 2 by means such as column chromatography. The amount of benzoyl chloride added dropwise is preferably 0.9 to 1.05 equivalents, which can control side reactions and provide benzoyl-mono-protected 2-butene-1,4-diol.

As the base used in Step 1 above, sodium hydride, potassium carbonate/dimethyl tin dichloride, etc. can be listed, with sodium hydride being preferred.

As the organic solvent used in the reaction of Step 1 above, tetrahydrofuran, N,N-dimethylformamide, chloroform, etc. can be listed.

Step 2, the asymmetric Simmons-Smith cyclopropanation reaction, can be performed as follows, but is not

limited thereto. Dissolve diethylzinc in an organic solvent at -20°C to -25°C, add ethylene glycol dimethyl ether, and slowly drop diiodomethane, then add Compound 2 and an asymmetric inducer, stir at room temperature until the starting material is consumed. Quench the reaction with a ammonium chloride solution, extract the reaction solution, combine the organic phases, dry and concentrate to obtain a residue. Separate and obtain Compound 3 by means such as column chromatography. The organic solvents used include but are not limited to dichloromethane or n-hexane, and the asymmetric inducers include but are not limited to chiral butyl borate or chiral sulfonamide ligands, preferably 2-butyl-N,N,N',N'-tetramethyl-1,3,2-dioxaborolane-(4R,5R)-dicarboxamide. The molar ratio of Compound 2 to diethylzinc and diiodomethane can be 1: (3-5): (6-10). Relative to Compound 2, the asymmetric inducer is preferably 1.05-1.1 equivalents.

Step 3 can be performed as follows, but is not limited thereto. Dissolve Compound 3 in an organic solvent in an ice bath, add Dess-Martin periodinane, and stir at room temperature until the starting material is consumed. Quench the reaction with sodium thiosulphate solution and sodium bicarbonate solution, extract the reaction solution, concentrate, separate and obtain Compound 4 by means such as column chromatography. The molar ratio of Compound 3 to Dess-Martin periodinane can be 1: (1.0-1.2), with a reaction time preferably ranging from 2 to 5 hours. The organic solvent is preferably dichloromethane.

Step 4, the Corey-Fuchs reaction, can be performed as follows, but is not limited thereto. Dissolve carbon tetrabromide in dichloromethane at -10°C to -20°C, and add a solution of triphenylphosphine in dichloromethane dropwise, cool down to -20°C to -78°C, add Compound 4, and monitor until the reaction is complete. Raise to room temperature, remove the solvent by vacuum distillation. Dissolve the residue in an ethanol solution with a volume concentration of 40%, add n-hexane and stir vigorously. Stand for layer separation and repeat 2-3 times, combine the n-hexane phase, concentrate under reduced pressure to provide Compound 5, which is used directly in the subsequent reaction without further purification. The molar ratio of Compound 4, triphenylphosphine and carbon tetrabromide can be, for example, 1:2:4.

Step 5, the debromination of dibromoalkenes, can be performed as follows, but is not limited thereto. Dissolve Compound 5 in an organic solvent, add a base, reflux until the starting material is consumed, and concentrate under reduced pressure, separate and obtain Compound 6 by means such as column chromatography. The organic solvent is preferably tetrahydrofuran or 1,4-dioxane, etc.. The base can be listed as tetrabutylammonium fluoride or its trihydrate, etc..

Step 6, the Cadiot-Chodkiewicz reaction, can be performed as follows, but is not limited thereto. Dissolve cuprous chloride in an aqueous solution of organic amine in an ice bath, add hydroxylamine hydrochloride, then add methyl 4-ethynylbenzoate and Compound 6, and stir at 0°C to room temperature until the reaction is complete. Extract the reaction solution, concentrate, separate and obtain Compound 7 by means such as column chromatography. The molar ratio of Compound 6 to cuprous chloride, organic amine, and hydroxylamine hydrochloride can be, for example, 1:0.5:(20-30):(0.7-1). The organic amine includes, but is not limited to, ethylamine, n-butylamine, etc., with n-butylamine being preferred. The mass concentration of the organic amine aqueous solution is preferably 30%.

Step 7, the hydrolysis reaction, can be performed as follows, but is not limited thereto. Dissolve Compound 7 in a mixed solution of tetrahydrofuran and methanol, add an aqueous solution of base, and stir at room temperature until the starting material is consumed. Add dilute hydrochloric acid to adjust the pH to 1-2, precipitate a solid, separate and obtain Compound 8 by vacuum filtration. The molar ratio of Compound 7 to base can be, for example, 1:(3-4). The reaction time can be 2-4 hours. The base includes, but is not limited to, lithium hydroxide or sodium hydroxide, etc., with sodium hydroxide being preferred.

Step 8, the methylation reaction, can be performed as follows, but is not limited thereto. Dissolve Compound 8 in an organic solvent in an ice bath, add a base, stir, then add methyl iodide, and monitor until the starting material is consumed. Add sodium hydroxide aqueous solution and monitor until the reaction is complete. Add dilute hydrochloric acid to adjust the pH to 1-2, extract the reaction solution, and concentrate to provide Compound 9. The organic solvent includes, but is not limited to, tetrahydrofuran or N,N-dimethylformamide, etc.. The molar ratio of Compound 8 to base and methyl iodide can be, for example, 1:(3-4):(3-4). The base is preferably sodium hydroxide.

Step 9, the condensation reaction, can be performed as follows, but is not limited thereto. Dissolve Compound 9, methyl (S)-2-amino-3-(tert-butoxycarbonylamino)-3-methylbutanoate, O-(7-azabenzotriazol-1-yl)-N,N,N',N'-tetramethyluronium hexafluorophosphate in an organic solvent, add an organic base, and stir at room temperature until the starting material is consumed. Extract the reaction solution, concentrate, separate and obtain Compound 10 by means such as column chromatography. The organic solvent includes, but is not limited to, dichloromethane or N,N-dimethylformamide, etc., and the organic base includes, but is not limited to, diisopropylethylamine or triethylamine, etc.. The molar ratio of Compound 9 to methyl (S)-2-amino-3-(tert-butoxycarbonylamino)-3-methylbutanoate, organic base, and O-(7-azabenzotriazol-1-yl)-N,N,N',N'-tetramethyluronium hexafluorophosphate can be 1:(1.1-1.3):(2-4):(1.1-1.3). The reaction time can be 3-8 hours.

Step 10, the deprotection reaction, can be performed as follows, but is not limited thereto. Dissolve Compound 10 in an organic solvent, add an acid, and stir at room temperature until the reaction is complete, and then concentrate to provide Compound 11. The organic solvent includes, but is not limited to, tetrahydrofuran, dichloromethane, diethyl ether,

ethyl acetate, chloroform, etc.. The acid includes, but is not limited to, hydrochloric acid or trifluoroacetic acid.

Step 11, the hydroxylaminolysis reaction, can be performed as follows, but is not limited thereto. Dissolve Compound 11 in an organic solvent, add a hydroxylamine aqueous solution, and stir at room temperature until the starting material is consumed, and then concentrate, the residue is separated and purified to provide compound Ia. The separation and purification of the residue can be conducted by reverse phase HPLC (conditions: chromatography column XDB-C18 (21.2 mm × 250 mm, 7 μm; mobile phase A: methanol, B: water (containing 0.1% TFA); gradient elution (0-40 min: A 10%-100%); column temperature 25°C; flow rate 8 mL/min; detection wavelength 280 nm), followed by freeze-drying to provide compound Ia. The organic solvent includes but is not limited to isopropanol, methanol, tetrahydrofuran, etc.. The molar ratio of Compound 11 to hydroxylamine in the hydroxylamine aqueous solution can be 1:(18-22). The concentration of the hydroxylamine aqueous solution can be, for example, 50% by mass.

More specifically, the present application provides a method for preparing the compound of Ia, which comprises the following steps.

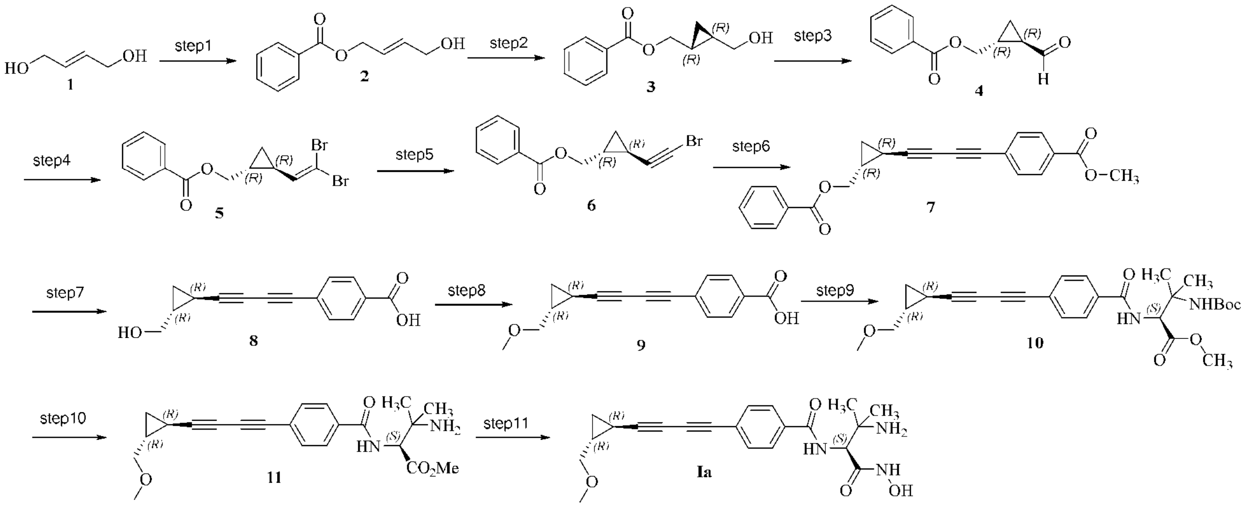


The conditions and operational methods for the above steps are as described above.

More specifically, the present application provides a method for preparing the compound of Ia, which comprises the following steps.

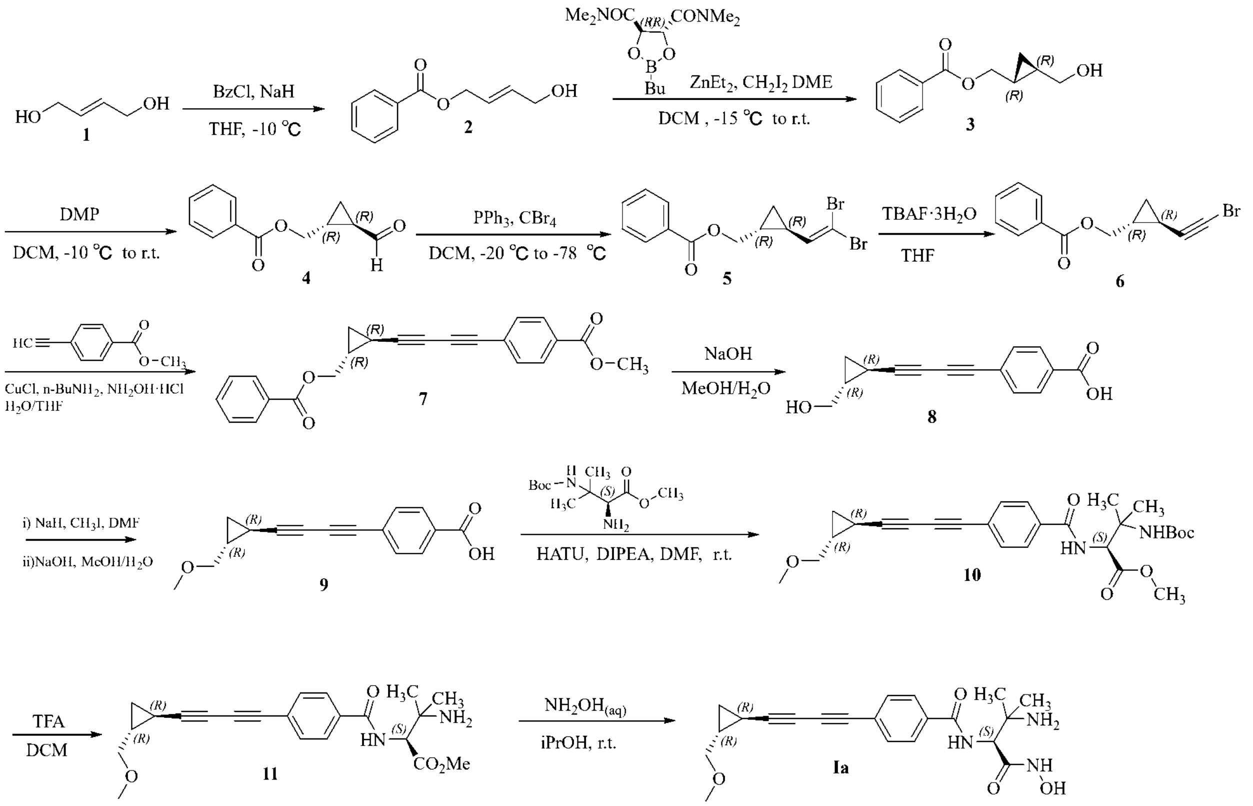


1 2 3 4 5 6 7 8 9 10 11 Ia

The compound of formula I can be prepared by using a *cis* and *trans* mixture of 2-butene-1,4-diol as raw material, without using an asymmetric inducer in step 2, and following the same preparation method as described above for the compound of formula Ia.

The racemate of formula Ia/Ib can be prepared by using *trans*-2-butene-1,4-diol as raw material, without using an asymmetric inducer in step 2, and following the same preparation method as described above for the compound of formula Ia.

The present invention further provides a method for preparing compounds of formula Ib, Ic and Id, which comprises:

(1) subjecting 2-butene-1,4-diol to an esterification reaction with benzoyl chloride under basic conditions to provide benzoyl-mono-protected 2-butene-1,4-diol;

(2) oxidising the benzoyl-mono-protected 2-butene-1,4-diol to provide an α,β-unsaturated aldehyde, which is then reacts with triethyl orthoformate to form acetaldehyde; subsequently reacts with optically active diisopropyl D-(-)-tartrate or diisopropyl L-(+)-tartrate, followed by Simmons-Smith cyclopropanation and hydrolysis to provide the highly optically active cyclopropane Compound 2-formylcyclopropylmethylene benzoate;

(3) subjecting the corresponding optical isomer 2-formylcyclopropylmethylene benzoate to a Corey-Fuchs reaction, debromination, Cadiot-Chodkiewicz reaction, hydrolysis, methylation, condensation with methyl (S)-2-amino-3-(tert-butoxycarbonylamino)-3-methylbutanoate, deprotection, and hydroxylaminolysis to provide the optical isomers Ib, Ic, and Id.

More specifically, the present invention provides a method for preparing the compound of Ib, Ic or Id, comprising one or more of the following steps:

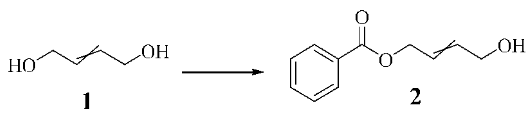

1 2

Step 1'

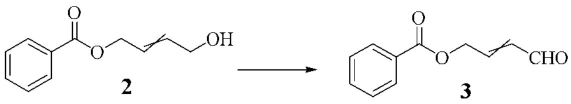

2 3

Step 2'

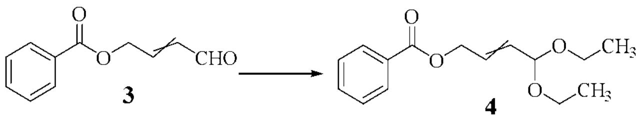

3 4

Step 3'

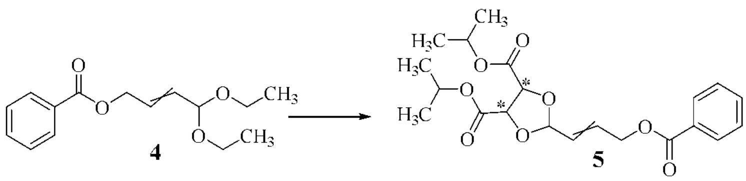

4 5

Step 4'

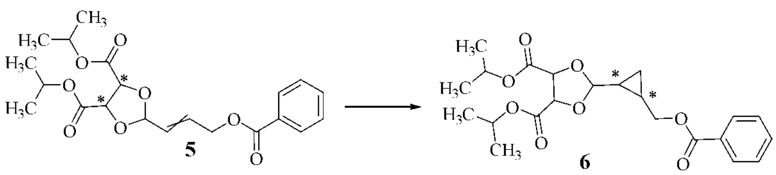

5 6

Step 5'

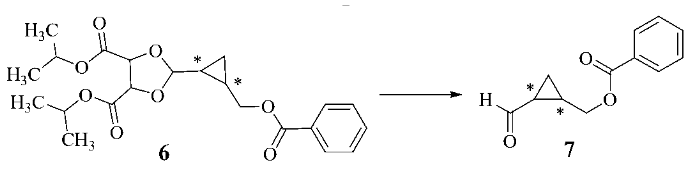

6 7

Step 6'

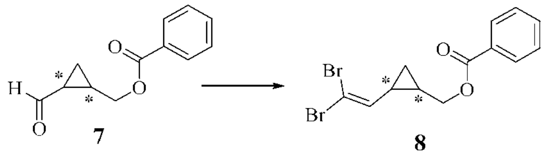

7 8

Step 7'

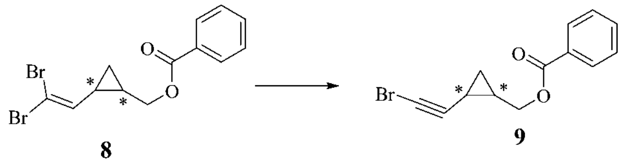

8 9

Step 8'

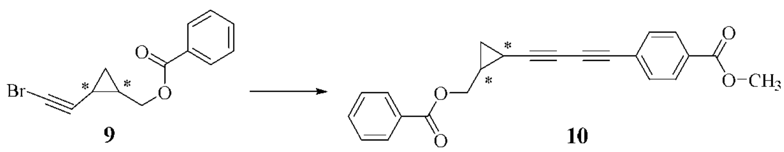

9 10

Step 9'

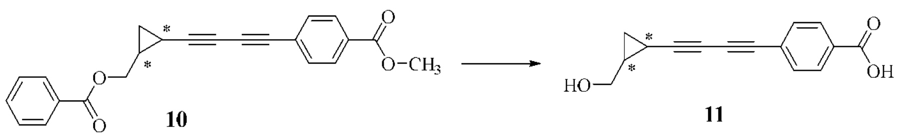

10 11

Step 10'

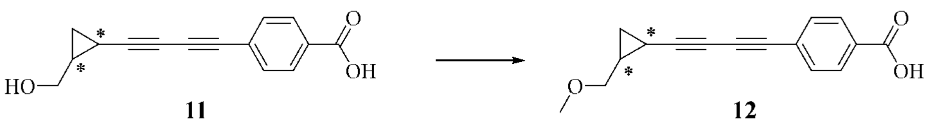

11 12

Step 11'

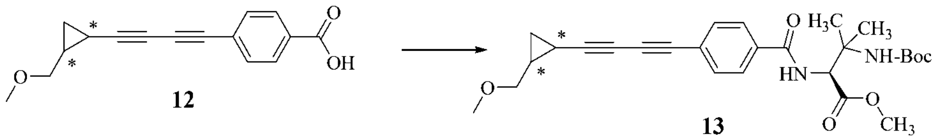

12 13

Step 12'

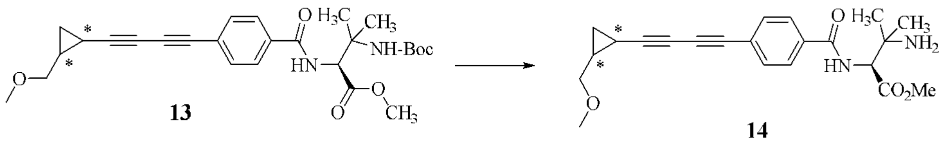

Step 13'

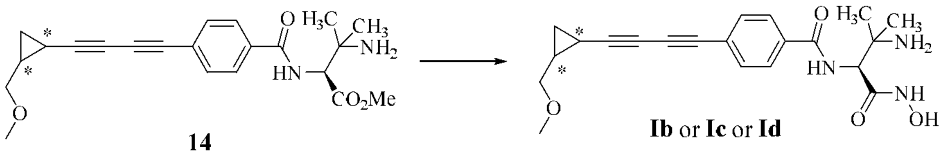

Step 14'.

Steps 1', 7', 8', 9', 10', 11', 12', 13', and 14' correspond one-to-one with steps 1, 4, 5, 6, 7, 8, 9, 10, and 11, respectively, in the preparation of the compound of formula Ia and can be carried out by the same method.

Step 2' can be performed as follows, but is not limited thereto. Dissolve Compound 2 in an organic solvent in an ice bath, add Dess-Martin periodinane, and stir at room temperature until the starting material is consumed. Quench the reaction with sodium thiosulphate solution and sodium bicarbonate solution, extract the reaction solution, concentrate, separate and obtain Compound 3 by means such as column chromatography. The molar ratio of Compound 2 to Dess-Martin periodinane is preferably 1:(1.0-1.2). The organic solvent is preferably dichloromethane.

Step 3' can be performed as follows, but is not limited thereto. Dissolve Compound 3 in an organic solvent, add a catalytic amount of ammonium nitrate and triethyl orthoformate, stir at room temperature until the starting material is consumed, concentrate under reduced pressure, separate and obtain Compound 4 by means such as column chromatography. The organic solvent includes, but is not limited to, anhydrous ethanol, anhydrous methanol, etc.. The molar ratio of Compound 3 to triethyl orthoformate and ammonium nitrate can be 1:(1.5-2):0.05.

Step 4' can be performed as follows, but is not limited thereto. Dissolve Compound 4 in an organic solvent, add diisopropyl tartrate of the corresponding configuration and a catalyst, stir until the starting material is consumed, concentrate, separate and obtain Compound 5 by means such as column chromatography. Diisopropyl tartrate of the corresponding configuration is diisopropyl L-(+)-tartrate or diisopropyl D-(-)-tartrate. The organic solvent is preferably toluene, and the catalyst is preferably pyridine p-toluenesulfonate. The stirring temperature can be, for example, 90°C.

Step 5', the Simmons-Smith cyclopropanation reaction, can be performed as follows, but is not limited thereto. Dissolve Compound 5 in an organic solvent at -15°C to -20°C, add a solution of diethylzinc in toluene dropwise, followed by a slow addition of diiodomethane, and stir at room temperature until the starting material is consumed. Quench the reaction with ammonium chloride solution, extract the reaction solution, combine the organic phases, dry, concentrate to provide the residue, separate and obtain Compound 6 by means such as column chromatography. The organic solvent used includes, but is not limited to, dichloromethane or n-hexane. The molar ratio of Compound 5 to diethylzinc and diiodomethane can be 1:(3-5):(6-10).

Step 6' can be performed as follows, but is not limited thereto. Dissolve Compound 6 in an acetic acid aqueous solution with a mass fraction of 70%~85%, preferably 80%, stir until the starting material is consumed, cool to room temperature, followed by a slow addition of sodium bicarbonate solution until no further bubbles were produced, then extract, concentrate, separate and obtain Compound 7 by means such as column chromatography. The stirring temperature can be, for example, 90°C.

More specifically, the present invention provides a method for preparing the compound of Ib, Ic or Id, which comprises the following steps:

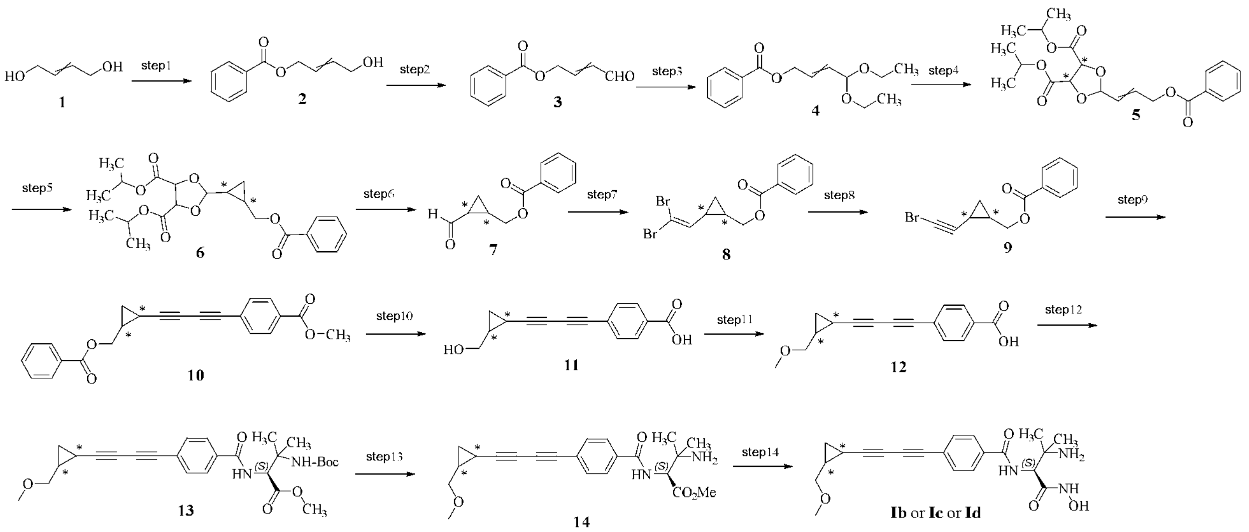


The pharmaceutical composition of the present invention comprises a compound of formula (1) or a pharmaceutically acceptable salt thereof and a pharmaceutically acceptable carrier, preferably comprises a compound of formula (2) or a pharmaceutically acceptable salt thereof and a pharmaceutically acceptable carrier, more preferably comprises a compound of formula (I) or a pharmaceutically acceptable salt thereof and a pharmaceutically acceptable carrier, and further preferably comprises one or more selected from compounds (Ia), (Ib),(Ic) and (Ib), and a pharmaceutically acceptable carrier, and particularly preferably comprises a compound of formula (Ia) or its pharmaceutically acceptable salt and a pharmaceutically acceptable carrier. The content of the above compound(s) in the pharmaceutical composition of the present invention is preferably 0.0001-99% by mass, more preferably 0.001-50% by mass, and further preferably 0.01-10% by mass.

Pharmaceutically acceptable carriers include various excipients conventionally used in pharmaceutical products or preparations, such as stabilizers, preservatives, dispersants, vehicles, etc..

The pharmaceutical composition of the present invention can be formulated into various preparations suitable for the active ingredient, including but not limited to oral preparations, injections, patches, suppositories, etc..

The present invention further provides the use of the above compounds or their pharmaceutically acceptable salts, or the above composition, for the manufacture of medicaments for treating infectious diseases caused by Gram-negative bacteria. Gram-negative bacteria can be exemplified by Escherichia coli, Klebsiella pneumoniae, Pseudomonas aeruginosa, Enterobacter cloacae, Enterobacter aerogenes, Serratia marcescens, Citrobacter freundii, Pantoea agglomerans, Proteus vulgaris, Proteus mirabilis, Shigella flexneri, etc..

The above compounds of the present invention or their pharmaceutically acceptable salts can be used in combination with other antimicrobial agents or active ingredients. Such combination use includes formulation into a composition with other antimicrobial agents or active ingredients, or administration concurrently or at intervals with other antimicrobial agents or active ingredients. The invention is further illustrated below with reference to examples, which should not be construed as limiting the scope of protection of the present invention. Any modification or alteration that does not depart from the spirit of the present invention falls within the scope of protection of the present invention.

Unless otherwise specified, reagents used in the following examples were commercially available.

**Example 1 Synthesis of N-(*S*)-3-amino-1-hydroxyamino-3-methyl-1-oxobutan-2-yl-4-[[(*1R,2R*)-2-(methoxymethylene)cyclopropyl]but-1,3-diynyl]benzamide (Ia)**

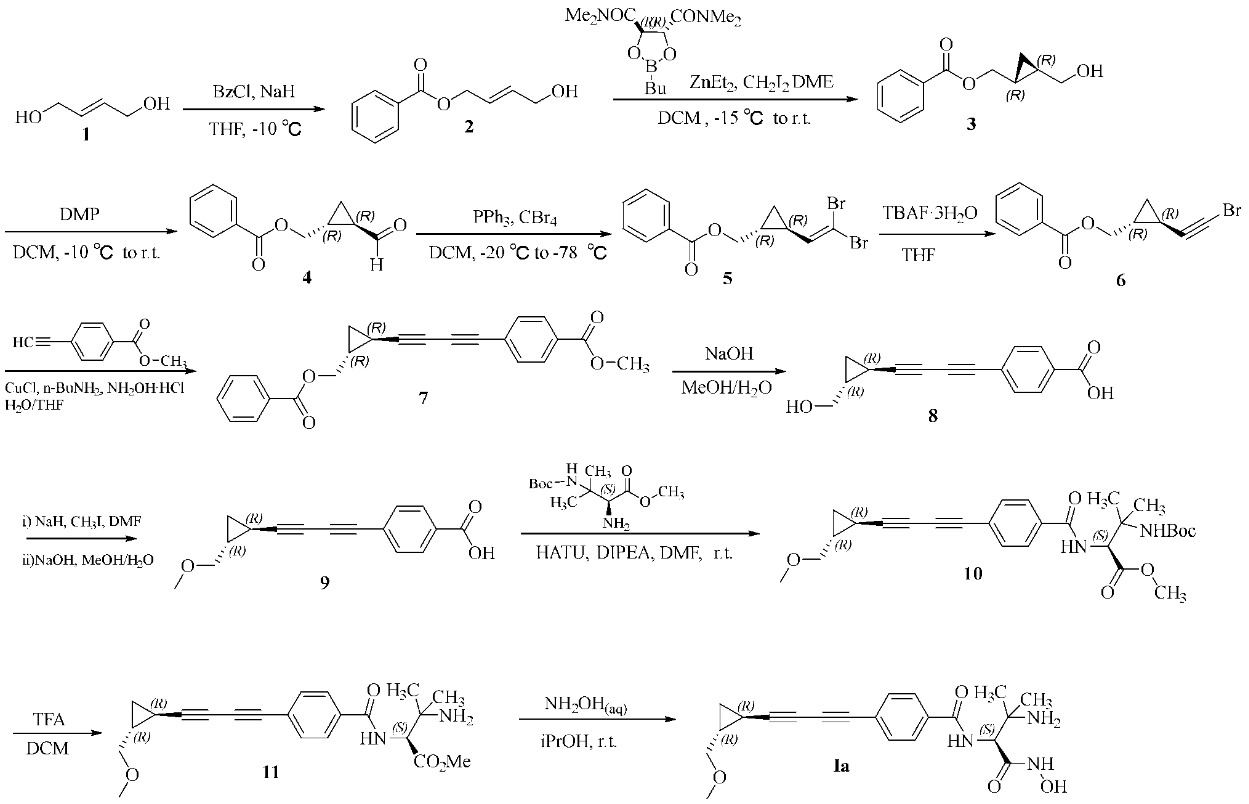


### *(E)*-4-Hydroxybut-2-enylbenzoate (Compound 2)

60% sodium hydride (90.80 g, 2.27 mol) was added in batches to a solution of Compound 1 (200.00 g, 2.27 mol) in THF (1.50 L) at -10°C, with gas generated and temperature risen, and the addition rate was controlled to keep the internal temperature below 0°C. After addition, the reaction mixture was stirred at the same temperature for 30 minutes, with the solution becoming gray and turbid, and becoming particularly viscous in large quantities. Benzoyl chloride (319.10 g, 2.27 mol) was added dropwise, and the internal temperature was controlled < 0°C. After addition, the reaction mixture was raised to room temperature and stirred for 3 hours. An appropriate amount of water was added to quench the reaction, and the reaction mixture was filtered to remove sodium hydride mineral oil, the filter cake was washed with THF, and the filtrate was concentrated under reduced pressure to obtain the crude product. The crude product was separated and purified by silica gel column chromatography [elution : petroleum ether : ethyl acetate (5:1-2:1)] to provide a pale yellow oily liquid (Compound 2) (227.00 g, 52.1%).

$^1$H NMR (600 MHz, $CDCl_3$): δ: 8.04 (dd, *J* = 8.2, 1.5 Hz, 2H), 7.56-7.52 (m, 1H), 7.42 (t, *J* = 7.8 Hz, 2H), 6.03-5.97 (m, 1H), 5.95-5.90 (m, 1H), 4.83-4.80 (m, 2H), 4.18 (dd, *J* = 5.0, 1.5 Hz, 2H), 2.13 (s, 1H).

MS-ESI(*m/z*): 215[M+Na]$^+$.

### Methyl ((*1R*,*2R*)-2-(hydroxymethyl)cyclopropyl)benzoate (Compound 3)

At -15°C under argon atmosphere, ethylene glycol dimethyl ether (CAS: 110-71-4; 30.52 g, 0.339 mol) was added into dichloromethane (300 mL) and stirred at the same temperature for 5 min. Diethylzinc (1 M n-hexane solution, 340 mL, 0.339 mol) was added in batches, with white fumes appeared above the liquid surface and then disappeared. After completion of the addition, the reaction mixture was stirred at the same temperature for 10 min. Under vigorous stirring, diiodomethane (182.13 g, 0.68 mol) was added dropwise, and the temperature was significantly increased. The dropwise addition rate was controlled to keep the internal temperature between - 15°C and -10°C. After completion of the addition, the reaction mixture was stirred at the same temperature for 30 min. A solution of 2-butyl-1,3,2-dioxaborolane-4S,5S-dicarboxylic acid bis(dimethylamide) (CAS: 161344-84-9; 20.26 g, 0.075 mol) in dichloromethane (100 mL) was added, followed immediately by the addition of a solution of Compound 2 (13.00 g, 0.068 mol) in dichloromethane (90 mL). After completion of the addition, the reaction mixture was stirred at the same temperature for 30 min. The cooling system was turned off and the temperature was slowly raised to room temperature, and stirred at room temperature (28°C) for 8 hours until reaction completion. The reaction solution was transferred to a 2 L single-neck flask and stirred for 10 minutes in a low-temperature reaction bath at 0°C. A saturated ammonium chloride aqueous solution (300 mL) was added in batches to

quench the reaction, with the temperature significantly increased. After completion of the addition, the reaction mixture was stirred for 10 minutes, and transferred to a separatory funnel. The aqueous layer was extracted with dichloromethane (300 mL × 3), the organic phases were combined, dried over anhydrous sodium sulfate, filtered, and concentrated under reduced pressure to obtain a yellow oily residue. A mixed solution of diethyl ether (300 mL) and water (200 mL) was added, and the two-phase solution was vigorously stirred at room temperature for 1 hour, and transferred to a separatory funnel, allowed to stand for separation of layers. The aqueous layer was extracted with diethyl ether (200 mL × 2), and the organic phases were combined and concentrated under reduced pressure to obtain approximately 250 mL of diethyl ether solution. The aqueous layer was used to recover the asymmetric inducer. At 0°C, a mixed solution of NaOH aqueous solution (2 N, 270 mL) and 30% $H_2O_2$ (45 mL) was added to the diethyl ether solution, stirred for 10 minutes, allowed to stand for separation of layers. The organic phase was washed with 10% HCl aqueous solution (220 mL), saturated sodium sulfite aqueous solution (220 mL), saturated sodium bicarbonate aqueous solution (220 mL), and saturated sodium chloride aqueous solution (220 mL), respectively. The organic phases were combined, dried over anhydrous sodium sulfate, filtered, and concentrated under reduced pressure to obtain a yellow oily product (Compound 3) (13.32 g, 95.1%). No further purification was required, and proceeded directly to the next reaction step. A small portion of the crude product was taken, and separated and purified by silica gel column chromatography [elution: petroleum ether: ethyl acetate (3:1)] to provide a colorless oily liquid for structural confirmation. Chiral HPLC purity: ee value 81.1%, chiral HPLC conditions: chromatographic column of Daicel CHIRACPAK®ID (ID00CE-VA036) (4.6 mm × 250 mm, 5 μm); mobile phase A: methanol, B: water, gradient elution (0-10 min: A 20%-100%); column temperature 25°C; flow rate 1 mL/min; detection wavelength 254 nm]; specific rotation $[\alpha]_{589}^{25} -14.60°$ (*c* = 1.00, $CH_3OH$).

$^1H$ NMR (600 MHz, $CDCl_3$): δ 8.07-8.03 (m, 2H), 7.55 (tt, *J* = 7.2, 1.4 Hz, 1H), 7.46-7.42 (m, 2H), 4.25-4.16 (m, 2H), 3.54 (dd, *J* = 11.4, 6.3 Hz, 1H), 3.49 (dd, *J* = 11.4, 6.7 Hz, 1H), 1.67 (s, 2H), 1.23-1.17(m, 2H), 0.66 (m, 2H).

$^{13}C$ NMR (126 MHz, $CDCl_3$): δ 166.81, 132.99, 130.36, 129.63, 128.41, 68.30, 65.92, 19.90, 15.75, 8.59.

MS-ESI(*m*/*z*): 229[M+Na]$^+$.

**Methyl ((*1R*,*2R*)-2-formylcyclopropyl)benzoate (Compound 4)**

At -10°C, Dess-Martin Periodinane (50.91 g, 120 mmol) was added in batches to a solution of Compound 3 (16.50 g, 80 mmol) in dichloromethane (300 mL), with the temperature risen markedly, the addition rate was controlled to keep the internal temperature between -7°C and -10°C. After completion of addition and temperature stabilization, the mixture was transferred to room temperature and stirred for 3 h, and then filtered with suction, the filter cake was washed with dichloromethane. The filtrate was successively neutralized to neutrality with saturated sodium bicarbonate solution, extracted and washed with saturated sodium thiosulphate aqueous solution (100 mL × 2) and saturated saline solution (100 mL). The residue was dried over anhydrous sodium sulphate, filtered, and concentrated under reduced pressure to provide the crude product, which was separated and purified by silica gel column chromatography (elution: petroleum ether : ethyl acetate = 30:1-10:1-5:1) to obtain a pale yellow oily product (Compound 4) (13.31 g, 81.5%).

$^1H$ NMR (600 MHz, $CDCl_3$): δ 9.18 (d, *J* = 4.5 Hz, 1H), 8.04-7.99 (m, 2H), 7.57-7.53 (m, 1H), 7.47-7.38 (m, 2H), 4.36-4.32 (m, 1H), 4.22-4.17 (m, 1H), 2.03-1.96 (m, 2H), 1.42-1.38 (m, 1H), 1.20-1.16 (m, 1H).

$^{13}C$ NMR (151 MHz, $CDCl_3$): δ 199.67, 166.27, 133.07, 129.76, 129.54, 128.33, 65.73, 28.01, 20.54, 12.65.

MS-APCI(m/z): 205 [M+H]$^+$.

**Methyl ((*1R*,*2R*)-2-(2,2-dibromovinyl)cyclopropyl)benzoate (Compound 5)**

At -20°C under argon atmosphere, the DCM (75 mL) solution of triphenylphosphine (68.40 g, 261 mmol) was added dropwise to the DCM (60 mL) solution of carbon tetrabromide (43.24 g, 130 mmol) (the two colorless solutions turned into a yellow liquid). After completion of dropping, the mixture was stirred at the same temperature for 30 minutes, and then cooled to -78°C. The DCM (60 mL) solution of Compound 4 (13.31 g, 65 mmol) was added dropwise. After completion of dropping, the mixture was stirred at the same temperature for 30 min, and then naturally raised to room temperature and stirred for a further 40 min. The solvent was evaporated and removed under reduced pressure. The residue was dissolved in 40% ethanol solution (400 mL) to obtain a yellow liquid. N-hexane was added and stirred vigorously for 10 minutes. The mixture was allowed to stand for layer separation, and repeated twice. The n-hexane layer was concentrated under reduced pressure, filtered with suction, and the filter cake was washed with n-hexane, and concentrated to obtain a yellow liquid, which was separated and purified by silica gel column chromatography (elution: petroleum ether : ethyl acetate = 150:1) to provide a pale yellow solid (Compound 5) (10.1 g, 43.0%).

$^1H$ NMR (600 MHz, $CDCl_3$): δ 8.08-8.04 (m, 2H), 7.59-7.54 (m, 1H), 7.47-7.42 (m, 2H), 5.88 (d, *J* = 9.0 Hz, 1H), 4.27 (dd, *J* = 11.6, 6.9 Hz, 1H), 4.19 (dd, *J*= 11.6, 7.3 Hz, 1H), 1.76-1.70 (m, 1H), 1.51-1.44 (m, 1H), 0.99 (dt, *J* = 8.6, 5.4 Hz, 1H), 0.87 (dt, *J* = 8.6, 5.2 Hz, 1H).

$^{13}C$ NMR (151 MHz, $CDCl_3$): δ 166.47, 139.84, 132.95, 130.17, 129.61, 128.34, 87.09, 67.26, 21.16, 19.22,

12.12.

MS-ESI(*m*/*z*): 381[M+Na]$^+$.

**Methyl ((*1R*, *2R*)-2-(2-bromoethynyl)cyclopropyl)benzoate (Compound 6)**

Compound 5 (10.10 g, 28 mmol) was dissolved in 250 mL tetrahydrofuran. Tetrabutylammonium fluoride trihydrate (47.89 g, 152 mmol, the amount added must be greater than 3.0 eq) was added, yielding a brown solution, and stirred at 70°C for 5 h. The reaction solution was concentrated, 200 mL ethyl acetate was added, washed with water and then with saturated sodium chloride solution, concentrated, and purified by column chromatography (elution: petroleum ether: ethyl acetate = 20:1) to provide a yellow oily product Compound 6 (5.57 g, 71.1%).

**Methyl 4-(((*1R*,*2R*)-2-((benzoyloxy)methyl)cyclopropyl)but-1,3-diynyl)benzoate (Compound** 7)

Under an ice bath, cuprous chloride (0.83 g, 8.34 mmol) was added to a 30% aqueous solution of n-butylamine (24.40 g, 333.6 mmol) to form a deep blue solution; hydroxylamine hydrochloride (0.81 g, 11.68 mmol)was added, whereupon the blue colour faded and the solution turned to pale yellow; a tetrahydrofuran solution of methyl 4-ethynylbenzoate (2.67 g, 17 mmol) was added to precipitate a solid, and then a tetrahydrofuran solution of Compound 6 (5.57 g, 20 mmol) was immediately added to provide a yellow turbid solution, which was stirred at room temperature overnight, filtered with suction, and the filter cake was rinsed with ethyl acetate. The sample was dry-loaded, and purified by column chromatography (elution: petroleum ether: ethyl acetate = 100:1-50:1-30:1) to provide a yellow oil product (Compound 7) (2.87 g, 48.0%).

$^1$H NMR (600 MHz, $CDCl_3$): δ 8.08-8.02 (m, 2H), 7.96 (d, *J* = 8.5 Hz, 2H), 7.60-7.54 (m, 1H), 7.52-7.49 (m, 2H), 7.45 (t, *J* = 7.8 Hz, 2H), 4.32-4.22 (m, 1H), 4.17-4.08 (m,1H), 3.91 (s, 3H), 1.80-1.74 (m, 1H), 1.57-4.52 (m, 1H), 1.18-1.14 (m, 1H), 1.08-1.00 (m, 1H), 0.98-0.85 (m, 1H).

$^{13}$C NMR (151 MHz, $CDCl_3$): δ 166.42, 166.32, 133.11, 132.42, 129.67, 129.46, 128.41, 87.19, 73.87, 66.76, 66.59, 61.47, 52.26, 21.65, 21.22, 14.09, 13.73, 6.59, 6.27.

MS-ESI(m/z):381 [M+H]$^+$.

**4-[[(*1R*,*2R*)-2-(hydroxymethyl) cyclopropyl]but-1,3-diynyl)benzoic acid (Compound 8)**

A solution of sodium hydroxide (1.28 g, 32 mmol) in water (10 mL) was added to a solution of Compound 7 (2.87 g, 8 mmol) in THF/MeOH (10/10 mL). The mixture was stirred at room temperature for 2 hours. The solvent was evaporated and removed under reduced pressure. Water (50 mL) was added to the concentrate, and extracted twice with dichloromethane (20 mL × 2). The aqueous phase was adjusted to approximately pH 2.0 with 1 mol/L hydrochloric acid, then extracted with ethyl acetate (50 mL × 4). The organic phases were combined, dried over anhydrous sodium sulphate, filtered, and concentrated under reduced pressure to provide an off-white solid (Compound 8) (1.54 g, 80.2%), which was used directly in the subsequent reaction without further purification.

Compound 8 purity 99.51%, retention time 9.183 min [HPLC conditions: chromatographic column SB-$C_{18}$ (4.6 mm × 150 mm, 5 μm; mobile phase A: methanol (containing 1%o TFA), B: water (containing 1‰ TFA), gradient elution (0-10 min: A 20%-100%); column temperature 25°C; flow rate 1 mL/min; detection wavelength 280 nm]. Chiral HPLC purity: main peak retention time 7.697 min, content 98.05%; isomer 1 retention time 6.255 min, content 0.40%; isomer 2 retention time 6.506 min, content 0.99% [chiral HPLC conditions: chromatographic column Daicel CHIRACPAK®ID (ID00CE-VA036) (4.6 mm × 250 mm, 5 μm); mobile phase A: ethanol (containing 1%o TFA), B: n-hexane (containing 1%o TFA), gradient elution (0-10 min: A 20%-100%); column temperature 25°C; Flow rate 1 mL/min; detection wavelength 280 nm], e.e. value 99.2%. Specific rotation [ $[\alpha]_{589}^{25}-103.68^{\circ}$ (*c* 0.453, $CH_3OH$)]; Optical rotation measurement method: the compound was precisely weighed, placed in a 10 mL volumetric flask, dissolved in analytical-grade methanol, diluted to the 10 mL mark, and measured with a Rudolf polarimeter using the corresponding blank solvent as control.

mp 181.4-183.6 °C.

$^1$H NMR (600 MHz, DMSO-$d_6$): δ: 7.93 (dd, *J* = 16.8, 7.7 Hz, 2H), 7.62 (d, *J* = 7.7 Hz, 2H), 7.50 (t, *J* = 7.5 Hz, 1H), 2.02-1.96 (m, 1H), 1.48-1.44 (m,1H), 1.23 (d, *J* = 6.3 Hz, 3H), 1.03-0.78 (m, 2H).

MS-ESI(*m*/*z*): 239[M-H]$^-$.

To determine the absolute configuration of the constructed chiral ternary ring, Compound 8 was first esterified in the presence of sulfuric acid and methanol to provide Compound 8-1, thereby eliminating the influence of carboxylic acid hydrogen bonds on the dominant conformation in solution. Subsequently, a similar chromophore, 1,3-dienylphenyl, was introduced at the hydroxyl position through a condensation reaction to provide Compound 8-2, theoretically generating negative chirality. The actual chirality was determined by circular dichroism spectroscopy.

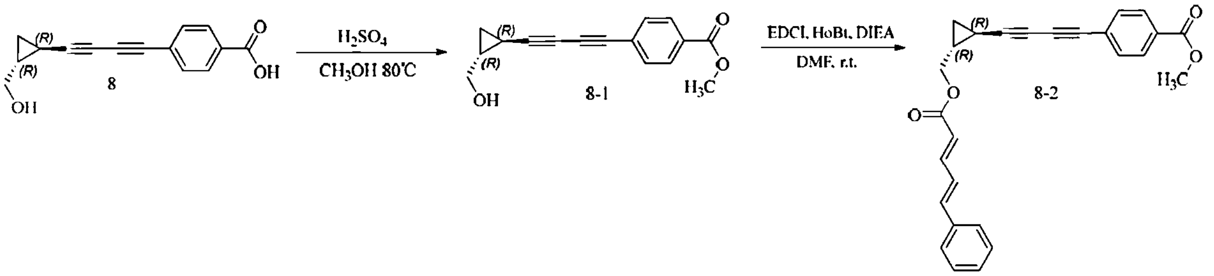

8 8-1 8-2

Circular dichroism measurement (ECCD) was performed using a Japanese-made JASCO J-815 circular dichroism spectrometer to measure the spectrum at 200-450 nm at room temperature. The concentration of the sample was 0.133% (w/v, methanol), with a 1 mm optical path length cuvette, 2.00 nm bandwidth, and a scanning speed of 100 nm/min. The ECCD results (shown in Figure 1) were consistent with the theoretical chirality.

Additionally, 30 mg of Compound 8 was weighed and dissolved in 50 mL of dichloromethane, left to evaporate naturally at 0-10 °C, yielding single crystals of Compound 8. A single crystal X-ray diffractometer was used to determine that the single crystals existed as dimers due to strong hydrogen bonding between the carboxyl groups. The single crystal results (shown in Figure 2) indicate that its cyclopropyl group is in the R,R configuration, consistent with the circular dichroism results, thereby determining the absolute configuration of the two chiral centers on the ternary ring.

**5-(((*1R*,*2R*)-2-(methoxymethylene)cyclopropyl)but-1,3-diynyl)benzoic acid (Compound 9)**

Under an ice bath, 60% sodium hydride (1.26 g, 31.5 mmol) was added in batches to a DMF (20 mL) solution of Compound 8 (2.52 g, 10.5 mmol), stirred at the same temperature for 40 min. Iodomethane (7.45 g, 52.5 mmol) was slowly added dropwise to the reaction solution. After temperature stabilization, the mixture was removed from the ice bath and stirred at room temperature under dark conditions for 10 h. Under an ice bath, a saturated ammonium chloride aqueous solution was added dropwise to quench the reaction, ethyl acetate (200 mL) was added, washed with water (120 mL × 3), the organic phase was dried over anhydrous sodium sulphate, filtered, and concentrated under reduced pressure to obtain a yellow oily liquid, 2.61 g, 97.7% yield, MS-ESI(m/z): 269[M+H]$^+$; no further purification required, proceeded directly to the next reaction step.

An aqueous solution (6 mL) of sodium hydroxide (1.16 g, 29.1 mmol) was added to the above yellow oily liquid (2.61 g, 9.7 mmol) in THF/MeOH (6 mL/6 mL), stirred at room temperature for 3 h. The solvent was evaporated and removed under reduced pressure. Water (5 mL) was added to the concentrate, and the pH was adjusted to approximately 3.0 with 1 mol/L hydrochloric acid, extracted with ethyl acetate (50 mL × 4), and the organic phases were combined, dried over anhydrous sodium sulphate, filtered, and concentrated under reduced pressure to obtain yellow solid Compound 9 (2.03 g, 82.5%), MS-ESI(m/z): 253[M-H] ; used directly in subsequent reaction without purification.

**Methyl (*S*)-2-14- [[(*1R*,*2R*)-2-(methoxymethylene)cyclopropyl]but-1,3-diynyl]benzamido]-3-(tert-butoxycarbonylamino)-3-methylbutanoate (Compound 10)**

Methyl (*S*)-2-amino-3-(tert-butoxycarbonylamino)-3-methylbutanoate (1.43 g, 5.81 mmol, purchased from Bide Pharmaceutical, purity: 95%) and diisopropylethylamine (DIPEA, 1.50 g, 11.62 mmol) were added to a DCM (15 mL) solution of Compound 9 (1.48 g, 5.81 mmol) and O-(7-azabenzotriazol-1-yl)-N,N,N',N'-tetramethyluronium hexafluorophosphate (HATU, 2.65 g, 6.97 mmol). The mixture was stirred at room temperature for 4 hours. The reaction solution was washed twice with water, once with saturated sodium chloride solution, dried over anhydrous sodium sulphate, filtered, and concentrated under reduced pressure to provide the crude product, which was separated and purified by silica gel column chromatography [elution: petroleum ether: ethyl acetate = 10:(1-1):1] to provide a yellow oily product (Compound 10) (2.70 g, 96.4%).

**Methyl (*S*)-3-amino-2-[4-[1(*1R*,*2R*)-2-(methoxymethylene)cyclopropyl]but-1,3-diynyl] benzamidol-3-methylbenzoate hydrochloride (Compound 11)**

Compound 10 (2.70 g, 5.60 mmol) was dissolved in dichloromethane (20 mL), to which 7 mL trifluoroacetic acid was added. The mixture was stirred at room temperature for 2 hours. The pH was adjusted to >8 with 2 N sodium hydroxide solution. The organic phase was washed with water, then with saturated sodium chloride solution, dried over anhydrous sodium sulphate, filtered, and the solvent was evaporated and removed under reduced pressure to obtain the crude yellow oily product (Compound 11) (2.16 g, 101%), which was used directly in the subsequent reaction without further purification.

**N-(S)-3-amino-1-hydroxyamino-3-methyl-1-oxobutan-2-yl-4-1[(1R,2R)-2-(methoxymethylene)cyclopropyl] but-1,3-diynyl]benzamide (Compound Ia)**

Under an ice bath, Compound 11 (2.16 g, 5.65 mmol) was dissolved in isopropanol (20 mL), to which a 50 mass% aqueous solution of hydroxylamine (11.3 mL) was added, and the mixture was stirred at room temperature for 24 hours, separated and purified by preparative liquid chromatography [mobile phase A: methanol, B: water, gradient elution (0-20 min: A 10%-100%; 25 min: A 100%; 26 min: A 10%; 30 min: A 10%); column temperature 25°C; flow rate 8 mL/min; detection wavelength 280 nm], and concentrated, dried, yielding a whitish solid (compound Ia) (0.92 g, 42.6%).

$^1$H NMR (400 MHz, DMSO-$d_6$): δ 11.23 (s, 1H), 9.21 (s, 1H), 8.82 (d, J = 9.3 Hz, 1H), 8.31 (s, 3H), 8.03 (d, J = 8.4 Hz, 2H), 7.61 (d, J = 8.4 Hz, 2H), 4.67 (d, J = 9.2 Hz, 1H), 3.33 (dd, J = 10.6, 5.4 Hz, 1H), 3.24 (s, 3H), 3.15 (dd, J = 10.7, 6.6 Hz, 1H), 1.58-1.45 (m, 2H), 1.36 (s, 3H), 1.33 (s, 3H), 1.05-0.97 (m, 1H), 0.95-0.84 (m, 1H).

$^{13}$C NMR (126 MHz, DMSO-$d_6$): δ 166.88, 165.54, 134.56, 132.18, 127.80, 123.65, 88.68, 76.23, 73.67, 73.25, 60.47, 58.36, 57.62, 51.82, 28.41, 27.20, 22.25, 13.34, 5.18.

Preparation of compound Ia trifluoroacetate: Compound Ia (2.17 g, 5.66 mmol) was dissolved in methanol (20 mL), cooled to -10°C, and 1 mL trifluoroacetic acid was added dropwise, with white fumes produced, and stirred at the same temperature for 2 hours, and then evaporated under reduced pressure, separated and purified by preparative liquid chromatography [mobile phase A: methanol, B: water, gradient elution (0-20 min: A 10%-100%; 25 min: A 100%; 26 min: A 10%; 30 min: A 10%); column temperature 25°C; flow rate 8 mL/min; detection wavelength 280 nm], and concentrated, dried, yielding a pale yellow solid (compound Ia trifluoroacetate) (1.76 g, 63%).

Preparation of compound Ia hydrochloride: Compound Ia was dissolved in methanol, cooled to - 5°C, and dry hydrogen chloride gas was injected into the above solution for 30 minutes, and then stirred at the same temperature for 1 h, and then evaporated under reduced pressure, separated and purified by preparative liquid chromatography [mobile phase A: methanol, B: water, gradient elution (0-20 min: A 10%-100%; 25 min: A 100%; 26 min: A 10%; 30 min: A 10%); column temperature 25°C; flow rate 8 mL/min; detection wavelength 280 nm], and concentrated, dried, yielding a pale yellow solid (compound Ia hydrochloride).

**Example 2 Synthesis of N-(*S*)-3-amino-1-hydroxyamino-3-methyl-1-oxobutan-2-yl-4-[[(*1S*,*2S*)-2-(methoxymethylene)cyclopropyl]but-1,3-diynyl]benzamide (Compound Ib)**

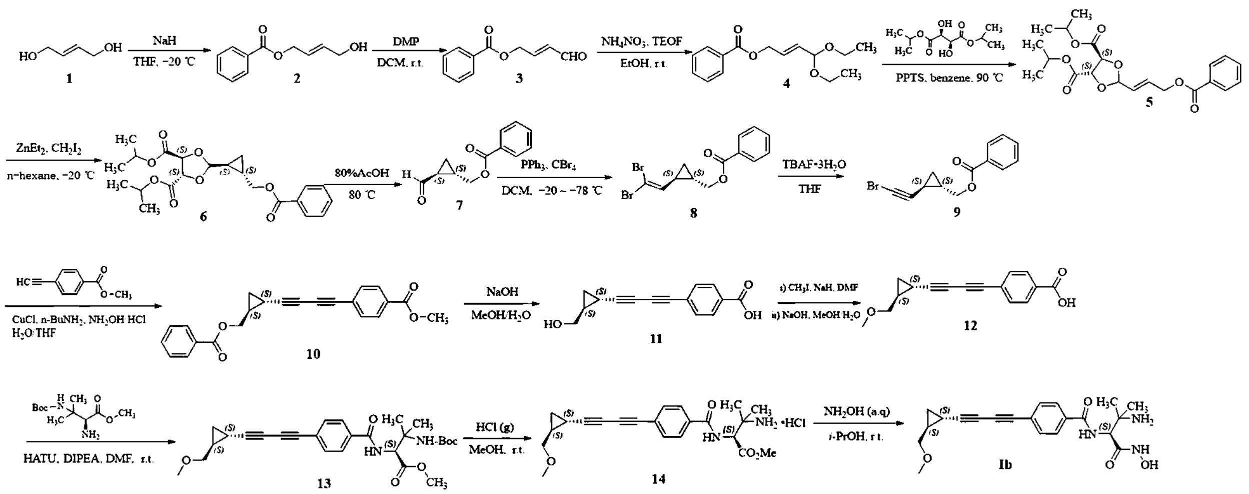


**(*E*)-4-hydroxybut-2-enylbenzoate (Compound 2)**

At -10°C, 60% sodium hydride (90.80 g, 2.27 mol) was added in batches to a solution of Compound 1 (200.00 g, 2.27 mol) in THF (1.50 L), with gas generated and temperature risen, and the addition rate was controlled to keep the internal temperature below 0°C. After addition, the reaction mixture was stirred at the same temperature for 30 minutes, with the solution becoming gray and turbid, and becoming particularly viscous in large quantities. Benzoyl chloride (319.10 g, 2.27 mol) was added dropwise, and the internal temperature was controlled < 0°C. After addition, the reaction mixture was raised to room temperature and stirred for 3 hours. An appropriate amount of water was added to quench the reaction, and the reaction mixture was filtered to remove sodium hydride mineral oil, the filter cake was washed with THF, and the filtrate was concentrated under reduced pressure to obtain the crude product. The crude product was separated and

purified by silica gel column chromatography [elution: petroleum ether : ethyl acetate (5:1-2:1)] to provide a pale yellow oily liquid (Compound 2) (227.00 g, 52.1%).

**(*E*)-4-oxobut-2-enylbenzoate (Compound 3)**

At 0°C, DMP (25.02 g, 59.0 mmol) was added in batches to a solution of Compound 2 (10.30 g, 53.6 mmol) in dichloromethane (DCM, 100 mL), the internal temperature was kept between -5°C and 0°C. After addition, the reaction mixture was stirred at room temperature for 4 hours. Saturated sodium thiosulphate solution and saturated sodium bicarbonate solution were added slowly, stirred for 30 minutes to quench the reaction, with a solid precipitated, and then filtered with suction. The filtrate was allowed to stand for separation of layers. The aqueous layer was extracted with dichloromethane (100 mL × 2), and the organic phases were combined, dried over anhydrous sodium sulphate, filtered, and concentrated under reduced pressure to provide the crude product, a pale yellow oily liquid (Compound 3) (11.60 g, >100%).

**(*E*)-4,4-diethoxybut-2-enylbenzoate (Compound 4)**

Ammonium nitrate (0.21 g, 2.63 mmol) was added to an ethanol (50 mL) solution of Compound 3 (11.60 g, 61.1 mmol) and triethyl orthoformate (11.90 g, 80.4 mmol), and stirred at room temperature for 15 hours, with the reaction solution changing from pale yellow to brown. The solvent was removed under reduced pressure. The concentrate was dissolved in ethyl acetate (150 mL) and successively washed with saturated sodium bicarbonate solution, water, saturated sodium chloride solution, dried over anhydrous sodium sulphate, filtered, and concentrated under reduced pressure to provide a brown oily product, which was separated and purified by silica gel column chromatography [elution: petroleum ether : ethyl acetate (5:1)] to provide a colorless oily liquid (Compound 4) (13.17 g, 85%).

**Diisopropyl (*4S*,*5S*,*E*)-2-(3-benzoyloxyprop-1-enyl)-1,3-dioxolane-4,5-dicarboxylate (Compound 5)**

Pyridinium p-toluenesulfonate (PPTS, 0.47 g, 1.9 mmol) was added to a solution of Compound 4 (9.89 g, 37.4 mmol) and diisopropyl D-(-)-tartrate (9.65 g, 41.2 mol, Bailingwei Technology, 99%) in toluene (50mL), heated to 90°C and reacted for 10 hours (the by-product ethanol was distilled off under reduced pressure). The reaction solution gradually changed from pale yellow to brown, cooled to room temperature, and concentrated under reduced pressure to provide the crude product, which was separated and purified by silica gel column chromatography [elution: petroleum ether : ethyl acetate (50:1-20:1 )] to provide a pale yellow oily liquid (Compound 5) (9.86 g, 65%).

**Diisopropyl (*4S*,*5S*)-2-[(*1S*,*2S*)-2-(benzoyloxymethyl)cyclopropyl]-1,3-dioxolane-4,5-dicarboxylate (Compound 6)**

At -20°C under argon atmosphere, a 2 mol/L toluene solution of diethylzinc (37 mL, 72.8 mmol) was added in batches to a n-hexane/dichloromethane (100/50 mL) solution of Compound 5 (9.86 g, 24.3 mmol). After addition, diiodomethane (38.99 g, 145.6 mmol) was added dropwise under vigorous stirring. After addition, the reaction mixture was raised to room temperature and stirred for 8 hours. Cold saturated ammonium chloride aqueous solution (200 mL) was added to quench the reaction. The mixture was extracted with dichloromethane (100 mL × 2), and the organic phases were combined and successively washed with saturated sodium thiosulphate solution, water, and saturated sodium chloride solution, dried over anhydrous sodium sulphate, filtered, and concentrated under reduced pressure to provide a pale yellow oily solid (Compound 6) (18.60 g, >100%). LCMS: $[421]^+$.

**[(1R,2R)-2-formylcyclopropyl]methyl acetate (Compound 7)**

Compound 6 (18.60 g, 44.2 mmol) was dissolved in 80% acetic acid aqueous solution (60 mL), heated to 90°C, and stirred for 5 hours. Cooled to room temperature, the reaction solution was dropped into saturated sodium bicarbonate solution. The mixture was extracted with DCM (80 mL × 3), and the combined organic phases were successively washed with water and saturated sodium chloride solution, dried over anhydrous sodium sulphate, filtered, and concentrated under reduced pressure to obtain crude product, which was separated and purified by silica gel column chromatography (elution P/E = 10/1-5/1) to provide a yellow oily liquid (Compound 7) (2.28 g, 25%).

**[(*1S*,*2S*)-2-(2,2- Dibromoethenyl)cyclopropyl]methylbenzoate (Compound 8)**

At -20°C under argon atmosphere, a DCM (30 mL) solution of triphenylphosphine (11.73 g, 44.7 mmol) was added dropwise to a DCM (20 mL) solution of carbon tetrabromide (7.41 g, 22.4 mmol) (the two colorless solutions turned

into a yellow liquid). After completion of the addition, the mixture was stirred at the same temperature for 30 min, and then cooled to -78°C and a DCM (10 mL) solution of Compound 7 (2.28 g, 11.2 mmol) was added dropwise. After completion of addition, the reaction solution was stirred at the same temperature for 30 min (the yellow solution turned to pink), and then gradually raised to room temperature. The solvent was evaporated and removed under reduced pressure to obtain the crude product, to which 200 mL of 40% ethanol solution was added, and extracted with n-hexane (80 mL × 3). The organic phases were combined, washed successively with water and saturated sodium chloride solution, concentrated, leaving a small amount of n-hexane, filtered with suction, and the filter cake was rinsed with ice-cold n-hexane, and the filtrate was concentrated to obtain a pale yellow oily product (Compound 8) (3.50 g, 87%).

The subsequent preparation method follows the corresponding steps in Example 1 to provide isomer Ib.

LCMS-ESI (m/z): 384.1[M+H]+.

$^1$H NMR (500 MHz, DMSO-$d_6$): δ 8.16 (s, 1H), 7.88 (d, *J* = 8.0 Hz, 2H), 7.63 (d, *J* = 8.0 Hz, 2H), 4.31 (s, 1H), 3.35 (dd, *J* = 10.6, 5.4 Hz, 1H), 3.26 (s, 3H), 3.17 (dd, *J* = 10.6, 6.6 Hz, 1H), 1.59-1.50 (m, 2H), 1.11 (s, 3H), 1.03 (s, 4H), 0.95-0.89 (m, 1H).

For 4-(((*1S*,*2S*)-2-(hydroxymethyl)cyclopropyl)but-1,3-diynyl)benzoic acid (Compound 11), the e.e. value is 98.4%, specific rotation [ $[\alpha]_{589}^{25}$ $+93.77^{\circ}$ (*c* 0.401, $CH_3OH$)]. Determination method: the compound was precisely weighed and placed in a 10 mL volumetric flask, dissolved in analytical-grade methanol, diluted to the 10 mL mark, and measured on a Rudolf polarimeter using the corresponding blank solvent as control. The Simmons-Smith cyclopropanation reaction proceeded via a synergistic mechanism, wherein a carbene-like compound was formed from $CH_2I_2$ and $Et_2Zn$, and then reacted with alkenes to form a ternary ring butterfly-shaped intermediate transition state. Consequently, in the absence of chiral auxiliaries, *trans-* or *cis*-alkenes underwent the Simmons-Smith cyclopropanation reaction to provide two different configurations of cyclopropane, which are enantiomers of each other. Thus, Compound 8 in Example 1 and Compound 11 in Example 2 were both synthesized from (*E*)-butene-1,4-diol via a chiral auxiliary reagent. The absolute configuration of Compound 8 was confirmed as (*1R, 2R),* and Compound 11 and Compound 8 were enantiomers of each other, with opposite signs but similar values of specific optical rotation, and a configuration of (*1S, 2S*).

### Example 3 Synthesis of N-(*S*)-3-amino-1-hydroxyamino-3-methyl-1-oxobutan-2-yl-4-[[(*1R*,*2S*)-2-(methoxymethylene)cyclopropyl]but-1,3-diynyl]benzamide (Compound Ic)

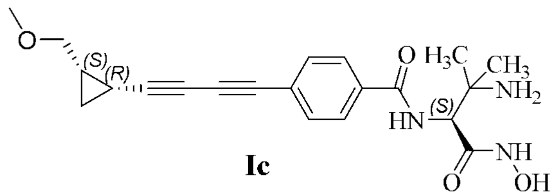

**Ic**

The preparation method is identical to that of Example 2, except that cis-2-butene-1,4-diol was used instead of trans-2-butene-1,4-diol as the starting material, and diisopropyl L-(+)-tartrate was employed as the chiral reagent for chemical separation. Compound Ic exhibits a purity of 98%.

LCMS-ESI (m/z): 384.3 [M+H]+.

$^1$H NMR (500 MHz, DMSO-$d_6$): δ 8.12 (s, 1H), 7.85 (d, *J* = 8.0 Hz, 2H), 7.61 (d, *J* = 8.0 Hz, 2H), 4.28 (s, 1H), 3.34 (dd, *J* = 10.6, 5.4 Hz, 1H), 3.24 (s, 3H), 3.15 (dd, *J* = 10.7, 6.6 Hz, 1H), 1.57-1.48 (m, 2H), 1.09 (s, 3H), 1.01 (s, 4H), 0.94-0.84 (m, 1H).

For the intermediate 4-[[(*1R*,*2S*)-2-(hydroxymethyl)cyclopropyl]but-1,3-diynyl]benzoic acid (Compound 11), e.e. value is 99.0%, specific optical rotation [ $[\alpha]_{589}^{25}$ $-133.33^{\circ}$ (*c* 0.180, $CH_3OH$)]. Determination method: the compound was precisely weighed and placed in a 10 mL volumetric flask, dissolved in analytical-grade methanol, diluted to the 10 mL mark, and measured on a Rudolf polarimeter using the corresponding blank solvent as control. Compound 11 in this example and Compound 11 in Example 4 were both synthesized from (Z)-butene-1,4-diol via a chiral auxiliary reagent, and being enantiomers of each other, with opposite signs but similar values of specific optical rotation. Compound 11 in Example 4 was confirmed by circular dichroism spectroscopy to be of (*1S,2R*) configuration, whereas Compound 11 in this example is of (*1R*,*2S*) configuration.

**Example 4 Synthesis of N-(*S*)-3-amino-1-hydroxyamino-3-methyl-1-oxobutan-2-yl-4-[[(*1S,2R*)-2-(methoxymethylene)cyclopropyl]but-1,3-diynyl]benzamide (Compound Id)**

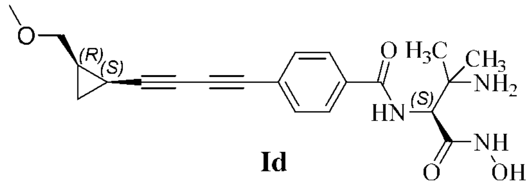

Id

The preparation method is identical to that of Example 2, except that *cis*-2-butene-1,4-diol was used instead of *trans*-2-butene-1,4-diol as the starting material, and diisopropyl D-(-)-tartrate was employed as the chiral reagent for chemical separation. Compound Id exhibits a purity of 98%.

LCMS-ESI (m/z): 384.3 $[M+H]^+$.

$^1H$ NMR (500 MHz, DMSO-$d_6$): δ 8.12 (s, 1H), 7.85 (d, *J* = 8.0 Hz, 2H), 7.61 (d, *J* = 8.0 Hz, 2H), 4.28 (s, 1H), 3.34 (dd, *J* = 10.6, 5.4 Hz, 1H), 3.24 (s, 3H), 3.15 (dd, *J* = 10.7, 6.6 Hz, 1H), 1.57-1.48 (m, 2H), 1.09 (s, 3H), 1.01 (s, 4H), 0.94-0.84 (m, 1H).

For the intermediate 4-[[(*1S*,*2R*)-2-(hydroxymethyl)cyclopropyl]but-1,3-diynyl]benzoic acid (Compound 11), e.e. value is 98.8%, specific rotation [ $[\alpha]_{589}^{25}$ $+115.93^{\circ}$ (c 0.590, $CH_3OH$)].

Determination method: the compound was precisely weighed and placed in a 10 mL volumetric flask, dissolved in analytical-grade methanol, diluted to the 10 mL mark, and measured on a Rudolf polarimeter using the corresponding blank solvent as control. Simultaneously, its configuration was determined according to the circular dichroism method used for Compound 8 in the preparation of Ia: theoretically resulting in positive chirality. Circular dichroism determination (ECCD) was performed using a JASCO J-815 circular dichroism spectrophotometer produced in Japan. The spectrum was measured at 200-400 nm at room temperature, and the concentration of the sample was 0.083% (w/v, methanol), with a 10 mm optical path length cuvette, 1.00 nm bandwidth, and a scanning speed of 100 nm/min. The ECCD results (shown in Figure 3) were consistent with the theoretical chirality, indicating that Compound 11 exhibits the (*1S,2R*) configuration, whereas its enantiomer Compound 11 in Example 3 adopts the (*1R,2S*) configuration.

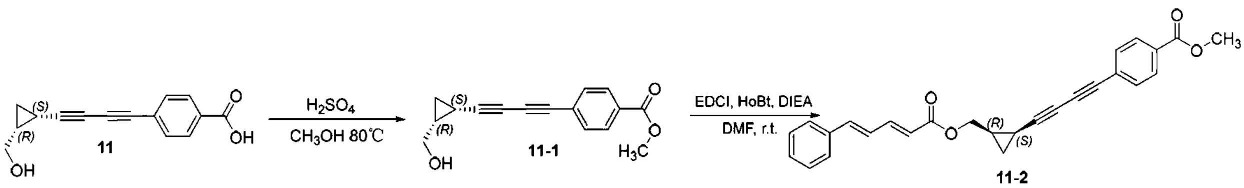


11 11-1 11-2

**Example 5 In vitro antibacterial activity**

According to the relevant operating procedures of the Clinical Laboratory Standards Institute (CLSI) in the United States, an antimicrobial susceptibility testing was conducted using the agar dilution method. A 1920 μg/mL stock solution was serially diluted twofold with sterile water to obtain dilutions of 960, 480, 240, 120, 60, 30, 15, 7.5, 3.75, 1.88, 0.94, 0.47 μg/mL, and 1 mL of the dilution was taken respectively from the dilutions with different concentrations and added to a Petri dish, to which 14 mL of melted MH agar medium was added and mixed thoroughly, and the final concentration of the drug in the petri dish is 128, 64, 32, 16, 8, 4, 2, 1, 0.5, 0.25, 0.12, 0.06, and 0.03 μ g/mL, respectively. The agar was allowed to solidify and set aside for later use.

The test bacteria were propagated in broth and adjusted to a turbidity of 0.5 McFarland (approximately 1-2 × $10^8$ CFU/mL) using a turbidimeter before inoculation. The 0.5 McFarland bacterial solution was then diluted 10-fold in broth (approximately 1-2 × $10^7$ CFU/mL). A multipoint inoculator was used to inoculate the above bacterial solution onto a series of plates containing different concentrations of drugs (with an inoculation amount of approximately $10^4$ CFU per inoculation site). After incubation at a constant temperature of 35°C for 18 hours, results were observed. The minimum concentration of drugs contained in sterile growth plates was determined as the Minimal Inhibitory Concentration (MIC). The results are shown in Table 1.

In Table 1, compound of formula (A) is the following compound disclosed in Chinese Patent Application CN110563611A:

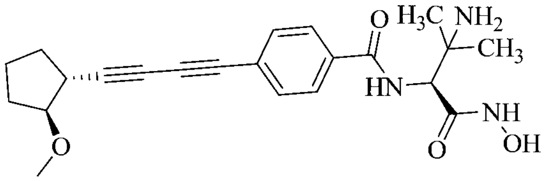

.

ACHN-975 is the following compound disclosed in Chinese Patent Application CN10329878A:

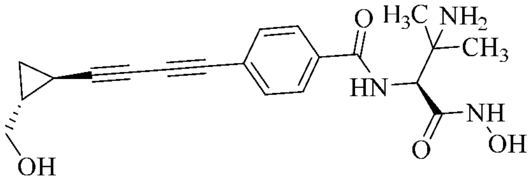

.

Table 1 In vitro antibacterial activity determination of 4 optical isomers and control drugs (MIC: μg/mL)

| Strain category | Strain number (resistant/enzyme-producing) | Compound of the present invention | | | | Control drugs | | | | |
|---|---|---|---|---|---|---|---|---|---|---|
| | | Ia | Ib | Ic | Id | A | Ia/Ib racemate | Formula I | ACHN-975 | Colistin |
| Escherichia coli | ATCC 35218 (ESBLs-producing) | 0.12 | 0.25 | 0.12 | 0.25 | 0.25 | 0.25 | 0.5 | 0.5 | 0.5 |
| | ATCC 2469 (CRE, NDM-1-producing) | 0.12 | 0.25 | 0.12 | 0.25 | 0.12 | 0.5 | 0.5 | 0.5 | 0.5 |
| | 13-43 (mcr-1) 17-R28 | 0.12 | 0.5 | 0.25 | 0.5 | 1 | 0.12 | 0.5 | 0.5 | 16 |
| | (CRE, NDM-1-producing) | 0.12 | 0.25 | 0.12 | 0.25 | 0.25 | 0.12 | 0.5 | 0.5 | 0.5 |
| Klebsiella pneumoniae | ATCC 700603 (ESBLs-producing) | 1 | 1 | 1 | 2 | 8 | 2 | 4 | 4 | 0.5 |
| | ATCC BAA-2146 (CRE, NDM-1-producing) | 1 | 2 | 2 | 2 | 8 | 2 | 8 | 4 | 1 |
| | 18-8 (ESBLs-producing) | 0.06 | 0.5 | 0.06 | 0.5 | 0.5 | 0.5 | - | 1 | 0.5 |
| | 17-R27 (CRE) | 0.5 | 0.25 | 0.5 | 0.5 | 1 | 1 | 2 | 2 | 16 |
| | 09-20 (mcr-1) | 0.25 | 0.5 | 0.25 | 0.5 | 1 | 0.5 | 2 | 1 | 16 |
| Pseudomonas aeruginosa | PAO1 | 0.5 | 1 | 0.5 | 1 | 2 | 1 | 2 | 0.25 | 2 |
| Enterobacter cloacae | CDC1000654 (NDM-1-producing) | 1 | 2 | 1 | 1 | 4 | 2 | 4 | 4 | 0.5 |
| Enterobacter aerogenes | ATCC 13048 | 0.5 | 0.5 | 0.5 | 0.5 | 0.5 | 1 | 2 | 1 | 0.5 |
| Serratia marcescens | ATCC 21074 | 0.25 | 0.25 | 0.25 | 0.25 | 0.25 | 0.25 | 0.5 | 0.25 | >128 |
| Citrobacter freundii | ATCC 43864 | 0.25 | 0.25 | 0.25 | 0.25 | 0.25 | 0.25 | 0.5 | 0.5 | 0.5 |
| Pantoea agglomerans | ATCC 27993 | 0.5 | 1 | 0.5 | 1 | 2 | 1 | 4 | 4 | 0.5 |

(continued)

| Strain category | Strain number (resistant/enzyme-producing) | Compound of the present invention | | | | Control drugs | | | | |
|---|---|---|---|---|---|---|---|---|---|---|
| | | Ia | Ib | Ic | Id | A | Ia/Ib racemate | Formula I | ACH-N-975 | Colistin |
| Proteus vulgaris | ATCC 29905 | 0.12 | 0.12 | 0.12 | 0.12 | 0.5 | 0.25 | 0.5 | 0.06 | >128 |
| Proteus mirabilis | ATCC 49565 | 0.25 | 1 | 0.5 | 1 | 1 | 1 | 2 | 2 | >128 |
| Shigella flexneri | ATCC 12022 | 0.06 | 0.12 | 0.06 | 0.06 | 0.25 | 0.12 | 0.25 | 0.12 | 0.5 |

Table 1 (continued)

| Strain category | Strain number (resistant/enzymeproducing) | Control drugs | | | | | | | |
|---|---|---|---|---|---|---|---|---|---|
| | | Aztreonam | Polymyxin B | Cefepime | Meropenem | Imipenem | Amikacin | Tigecycline | Levofloxacin |
| Escherichia coli | ATCC 35218 (ESBLs-producing) | ≤0.03 | 0.5 | ≤0.03 | ≤0.03 | 0.12 | 4 | 0.12 | ≤0.03 |
| | ATCC 2469 (CRE, NDM-1-producing) | 4 | 1 | 64 | 8 | 8 | >128 | 0.5 | 16 |
| | 13-43(mcr-1) | 16 | 8 | 2 | ≤0.03 | 0.12 | 4 | 1 | 64 |
| | 17-R28 (CRE, NDM-1-producing) | 8 | 0.5 | >128 | 128 | >128 | >128 | 0.12 | 8 |
| Klebsiella pneumoniae | ATCC 700603 (ESBLs-producing) | 16 | 1 | 1 | ≤0.03 | 0.25 | 1 | 8 | 1 |
| | ATCC BAA-2146 (CRE, NDM-1-producing) | >128 | 1 | 128 | 32 | 32 | >128 | 8 | >128 |
| | 18-8 (ESBLs-producing) | 1 | 1 | 1 | ≤0.03 | 0.12 | 1 | 2 | 1 |
| | 17-R27(CRE) | 0.25 | 16 | 0.5 | 0.25 | 0.12 | 2 | 2 | 16 |
| | 09-20(mcr-1) | 0.25 | 16 | 1 | ≤0.03 | 0.12 | >128 | 2 | 16 |
| Pseudomonas aeruginosa | PAO1 | 4 | 2 | 2 | 2 | 4 | 2 | 16 | 0.5 |
| Enterobacter cloacae | CDC1000654 (产 NDM-1) | >128 | 2 | 128 | 4 | 2 | >128 | 8 | 64 |
| Enterobacter aerogenes | ATCC 13048 | 0.12 | 1 | 0.06 | 0.06 | 0.25 | 1 | 1 | 0.06 |
| Serratia marcescens | ATCC 21074 | 0.12 | >128 | 0.06 | 0.06 | 0.5 | 2 | 2 | 0.25 |
| Citrobacter freundii | ATCC 43864 | 0.12 | 2 | ≤0.03 | ≤0.03 | 0.25 | 2 | 1 | ≤0.03 |
| Pantoea agglomerans | ATCC 27993 | ≤0.03 | 1 | 0.06 | ≤0.03 | 0.12 | 2 | 0.5 | 0.06 |
| Proteus vulgaris | ATCC 29905 | ≤0.03 | >128 | ≤0.03 | ≤0.03 | 0.25 | 1 | 2 | ≤0.03 |
| Proteus mirabilis | ATCC 49565 | ≤0.03 | >128 | 0.06 | 0.06 | 1 | 2 | 4 | 0.12 |

(continued)

| Strain category | Strain number (resistant/enzymeproducing) | Control drugs | | | | | | | |
|---|---|---|---|---|---|---|---|---|---|
| | | Aztreonam | Polymyxin B | Cefepime | Meropenem | Imipenem | Amikacin | Tigecycline | Levofloxacin |
| Shigella flexneri | ATCC 12022 | 0.06 | 1 | ≤0.03 | ≤0.03 | 0.12 | 4 | 0.12 | ≤0.03 |

It can be seen from the results in Table 1 that the four optical isomers Ia, Ib, Ic, and Id all have strong antibacterial effects on Gram-negative bacteria such as Escherichia coli, Klebsiella pneumoniae, Pseudomonas aeruginosa, Enterobacter cloacae, Enterobacter aerogenes, Serratia marcescens, Citrobacter freundii, Pantoea agglomerans, Proteus vulgaris, Proteus mirabilis, Shigella flexneri, etc., including clinically most resistant NDM-1-producing carbapenem-resistant Escherichia coli (CREC, NDM-1-producing), Klebsiella pneumoniae (CRKP, NDM-1-producing), superior to the compound of formula I. Overall, the antibacterial activity is superior to similar drugs ACHN-975, equivalent to or better than that of commonly used clinical drugs against Gram-negative bacteria. Among the four optical isomers, Ia and Ic have the strongest antibacterial activity.

**Example 6 In vivo antibacterial activity**

Mice were randomly grouped based on body weight, with 5 mice in each group, regardless of gender. The infectious bacteria was Escherichia coli ATCC BAA2469 (CRE, NDM-1-producing). The test bacterial solution was diluted with 5% high-activity dry yeast. Mice received intraperitoneal injection of 0.5 mL of 100% minimum lethal dose (100% MLD) bacterial solution. One intravenous administration was given at 1 hour post-infection at a dose volume of 10 mL/kg. Animal survival numbers were observed over 7 days, and survival rates were calculated. Results are shown in Table 2.

**Table 2 In vivo antibacterial activity test of 4 optical isomers and control drug against Escherichia coli ATCC BAA2469 intraperitoneal infection in mice**

| | Drug name | Dose (mg/kg) | Log dose (log mg/kg) | Number of animals (individuals) | Number of surviving animals (individuals) | Animal survival rate % |
|---|---|---|---|---|---|---|
| Compounds of the present invention | Ia | 1.4 | 0.15 | 5 | 4 | 80 |
| | | 0.7 | -0.15 | 5 | 2 | 40 |
| | | 0.35 | -0.46 | 5 | 0 | 0 |
| | Ib | 1.4 | 0.15 | 5 | 3 | 60 |
| | | 0.7 | -0.15 | 5 | 0 | 0 |
| | | 0.35 | -0.46 | 5 | 0 | 0 |
| | Ic | 1.4 | 0.15 | 5 | 4 | 80 |
| | | 0.7 | -0.15 | 5 | 2 | 40 |
| | | 0.35 | -0.46 | 5 | 0 | 0 |
| | Id | 1.4 | 0.15 | 5 | 3 | 60 |
| | | 0.7 | -0.15 | 5 | 0 | 0 |
| | | 0.35 | -0.46 | 5 | 0 | 0 |
| Comparative Examples | Ia/Ib racemate | 1.4 | 0.15 | 5 | 2 | 40 |
| | | 0.7 | -0.15 | 5 | 0 | 0 |
| | | 0.35 | -0.46 | 5 | 0 | 0 |
| | A | 6 | 0.78 | 5 | 1 | 20 |
| | | 3 | 0.48 | 5 | 0 | 0 |
| | | 1.5 | 0.18 | 5 | 0 | 0 |
| | ACHN-975 | 6 | 0.78 | 5 | 5 | 100 |
| | | 3 | 0.48 | 5 | 3 | 60 |
| | | 1.5 | 0.18 | 5 | 0 | 0 |
| | Colistin (converted) | 1.4 | 0.15 | 5 | 4 | 80 |
| | | 0.7 | -0.15 | 5 | 1 | 20 |
| | | 0.35 | -0.46 | 5 | 0 | 0 |
| | Control | — | — | 5 | 0 | 0 |

All four optical isomers Ia, Ib, Ic, and Id exhibit favorable in vivo antibacterial activity against NDM-1-producing carbapenem-resistant Escherichia coli in mice with intraperitoneal infection. Survival rates in the 1.4 mg/kg dose groups are 80%, 60%, 80%, and 60% respectively, markedly superior to the control drug ACHN-975. The in vivo therapeutic effects of Ia and Ic are similar and superior to those of Ib, Id and trans racemates. Compound A and ACHN-975 exhibit lower in vivo antibacterial activity than the other compounds, as detailed in Table 1. The 1.5 mg/kg dose groups for Compound A and ACHN-975 show 0% survival rate (i.e., 100% animal mortality rate) in mice with intraperitoneal infection by Escherichia coli ATCC BAA2469. The effective dose of in vivo antibacterial activity of Compound A is >3 mg/kg, and the effective dose of in vivo antibacterial activity of ACHN-975 is >1.5 mg/kg. In order to demonstrate the in vivo antibacterial activity of each compound, the dosages of Compound A and ACHN-975 were set at 1.5 mg/kg or above in this experiment. The effective doses of in vivo antibacterial activity for the other compounds (Ia, Ib, Ic, Id, Ia/Ib racemate and Colistin) are below 1.5 mg/kg, and consequently, the dosages for these compounds were set between 0.35 and 1.4 mg/kg (<1.5 mg/kg).

**Example 7 Acute toxicity in mice**

Healthy ICR strain mice weighing 20-21 g were taken for the experiment, randomly divided into groups of 3 mice

per group, regardless of gender. Each compound was administered via a single tail vein injection at a volume of 20 mL/kg. Animal responses were observed immediately postadministration and monitored for one week. The number of animal deaths was recorded and mortality rates calculated. Results are presented in Table 3.

**Table 3 Acute toxicity of 4 optical isomers and control drug in mice**

| | Test drug | Dose (mg/kg) | Log dose (log mg/kg) | Number of animals (individuals) | Number of deaths (individuals) | Mortality rate (%) |
|---|---|---|---|---|---|---|
| Compounds of the present invention | Ia | 200 | 2.30 | 3 | 3 | 100 |
| | | 150 | 2.18 | 3 | 0 | 0 |
| | Ib | 200 | 2.30 | 3 | 3 | 100 |
| | | 150 | 2.18 | 3 | 0 | 0 |
| | Ic | 200 | 2.30 | 3 | 3 | 100 |
| | | 150 | 2.18 | 3 | 1 | 33 |
| | | 100 | 2.00 | 3 | 0 | 0 |
| | Id | 200 | 230 | 3 | 3 | 100 |
| | | 150 | 2.18 | 3 | 2 | 67 |
| | | 100 | 2.00 | 3 | 0 | 0 |
| Comparative Examples | Ia/Ib racemate | 150 | 2.18 | 3 | 3 | 100 |
| | | 100 | 2.00 | 3 | 0 | 0 |
| | A | 200 | 2.30 | 3 | 3 | 100 |
| | | 150 | 2.18 | 2 | 0 | 0 |
| | ACHN-975 | 150 | 2.18 | 3 | 3 | 100 |
| | | 100 | 2.00 | 3 | 2 | 67 |
| | | 50 | 1.70 | 3 | 0 | 0 |

The preliminary acute toxicity of intravenous injection of four optical isomers Ia, Ib, Ic, and Id in mice is lower than that of the control drug ACHN-975. The mortality rates in the 150 mg/kg groups for Ia, Ib, Ic, Id, and ACHN-975 are 0%, 0%, 33%, 67%, and 100%, respectively. The toxicity of Ia and Ib is similar and weaker than that of Ic and Id. The mortality rate of the Ia/Ib racemate in the 150 mg/kg group is 100%, and the toxicity is greater than that of the individual isomers Ia and Ib.

**Example 8 Inhibition of LpxC enzyme activity in recombinant Escherichia coli**

Preparation of recombinant Escherichia coli LpxC enzyme (rLpxC). 2 μL of recombinant Escherichia coli LpxC enzyme (6.29 mg/mL) was added to 18 μL of distilled water. After thorough mixing, 2 μL of this mixture was added to 1042.4 μL of distilled water and mixed well to prepare a 36 nM working solution.

The substrate was UDP-3-O (R-hydroxytetradecanoyl)-N-acetylglucosamine, with UDP-α-D-N-Acetylglucosamine Disodium Salt as the internal standard. Mobile phase A: 90% water-10% acetonitrile containing 10 mM ammonium acetate; B: acetonitrile.

Compounds were diluted with distilled water to concentrations of 2560, 640, 160, 40, 10, 2.5, 0.625, 0.15625, 0.0390625, 0.009765625, 0.00244140625 nM.

Reaction system: Corresponding solutions were prepared as per the table below, and placed in a 37°C metal bath, and mixed at 300 rpm for 10 minutes. After mixing for 10 minutes, 10 μl of 250 μM substrate was added to each group to initiate the reaction, and placed in a 37°C metal bath and reacted at 300 rpm for 30 minutes.

| Category | Substrate group | Substrate + enzyme group | Substrate + enzyme + inhibitor group |
|---|---|---|---|
| 5 × reaction buffer [200 mM Hepes, 0.1% Brij L35] | 20 μL | 20 μL | 20 μL |
| water | 60 μL | 50 μL | 40 μL |
| 0.1% bovine serum albumin (BSA) | 10 μL | 10 μL | 10 μL |
| inhibitor | - | - | 10 μL |
| rLpxC enzyme 36 nM | - | 10 μL | 10 μL |

Termination solution: 1.1 μL of 1 mM internal standard was added to 2 mL of acetonitrile. 1.5 mL of EP tubes were taken and 100 μL of termination solution was added to each EP tube. The concentration of the internal standard in the final 350 μL system is 100 ng/mL.

Detection: 10 μL of reaction solution was added to 100 μL of the termination solution in an EP tube, mixed thoroughly, and then 240 μL of distilled water was added and mixed well, centrifuged at 14,000 rpm for 5 minutes. 70 μL of supernatant was transferred to an LC-MS vial and API 6500 Qtrap LC-MS system was used to measure substrates and metabolites. The median inhibition concentrations ($IC_{50}$) of the four optical isomers and ACHN-975 against rLpxC enzyme were calculated. Results are presented in Table 4.

Table 4 Inhibitory activity of 4 optical isomers and control drug against recombinant Escherichia coli LpxC enzyme

| Inhibitory activity | Compounds of the present invention | | | | Comparative example | | |
|---|---|---|---|---|---|---|---|
| | Ia | Ib | Ic | Id | Ia/Ib racemate | A | ACHN-975 |
| $IC_{50}$ (nM) | 1.072 | 0.8568 | 0.9047 | 0.8920 | 1.032 | 4.677 | 1.074 |

The four optical isomers Ia, Ib, Ic, Id, and ACHN-975, as well as Ia/Ib racemate exhibit similar inhibitory activity against the rLpxC enzyme.

**Example 9 Vero cytotoxicity**

Logarithmic phase Vero cells were taken, washed with PBS, and 2 mL trypsin was added, and then incubated in a 37°C incubator for 2 minutes to digest the cells. 3 mL of 1640 cell culture medium containing 10% FBSD was added and mixed thoroughly to terminate digestion. All liquid was transferred to a 15 mL centrifuge tube and centrifuged at 800 rpm for 5 min. The supernatant was discarded, and the residue was resuspended in 5mL of 1640 cell culture medium containing 10% FBS. A cell counter was used to measure cell density. Cells were inoculated at 100 μL and a density of $3 \times 10^5$/mL into a 96-well plate, with 6 replicates per group, and incubated at 37°C for 4 hours to allow cells to adhere to the wall.

The compound was twofold continuously diluted with PBS, and 10 μL of the compound with the corresponding concentration was added to the 96-well plate, and shaken and mixed well on a shaker, and then incubated at 37°C for 24 hours.

10 μL of CCK-8 reagent was added to the 96-well plate, mixed thoroughly on a shaker, and incubated at 37°C for 2 hours. A microplate reader was used to measure the absorbance at 450 nm wavelength, and the inhibition rate of the compound on Vero cells was calculated. Results are shown in Table 5.

Table 5 Vero cytotoxicity of 4 optical isomers and control drug

| Vero cytotoxicity | Compounds of the present invention | | | | Comparative examples | | |
|---|---|---|---|---|---|---|---|
| | Ia | Ib | Ic | Id | Ia/Ib racemate | A | ACHN-975 |
| $IC_{50}$ (μM) | > 128 | > 128 | > 128 | > 128 | > 128 | >64 | > 128 |

The four optical isomers and ACHN-975, Ia/Ib racemate exhibit negligible toxicity to Vero cells, with no detectable $IC_{50}$ (>128 μM).

## Claims

1. A compound as shown in the following formula (1) or a pharmaceutically acceptable salt thereof,

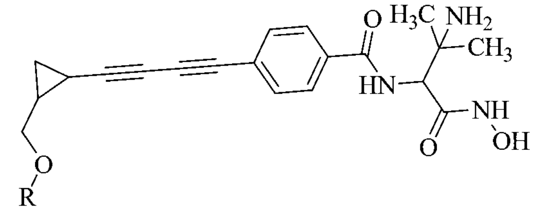

(1)

wherein R is $C_{1-6}$ alkyl.

2. The compound or the pharmaceutically acceptable salt thereof according to claim 1, which is the compound or the pharmaceutically acceptable salt thereof as shown in the following formula (2),

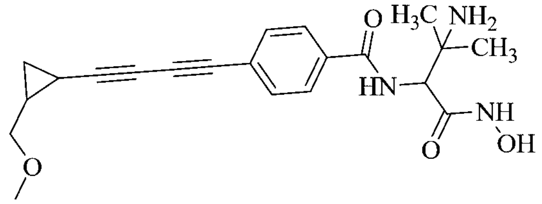

(2).

3. The compound or the pharmaceutically acceptable salt thereof according to claim 2, which is the compound or the pharmaceutically acceptable salt thereof as shown in the following formula (I),

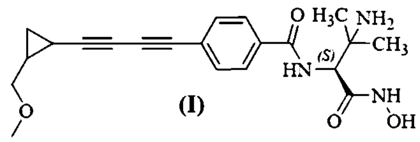

(I)

.

4. The compound or the pharmaceutically acceptable salt thereof according to claim 3, which is the compound or the pharmaceutically acceptable salt thereof as shown in any one of the following formula (Ia) to (Id),

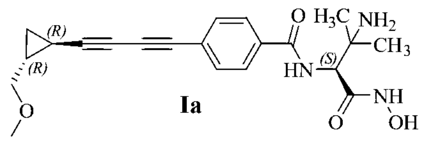

Ia

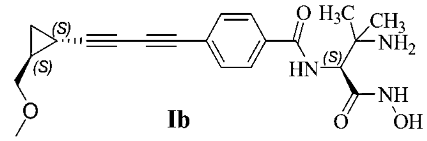

Ib

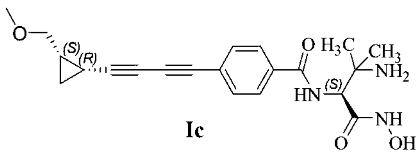

Ic

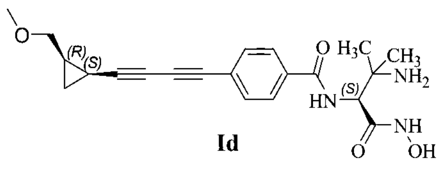

Id

.

5. A pharmaceutical composition comprising the compound or the pharmaceutically acceptable salt thereof according to any one of claims 1 to 4 and a pharmaceutically acceptable carrier.

6. The use of the compound or the pharmaceutically acceptable salt thereof according to any one of claims 1 to 4 for preparing a drug against Gram-negative bacteria.

7. The use according to claim 6, wherein the Gram-negative bacteria is selected from one or more of Escherichia coli,

Klebsiella pneumoniae, Pseudomonas aeruginosa, Enterobacter cloacae, Enterobacter aerogenes, Serratia marcescens, Citrobacter freundii, Pantoea agglomerans, Proteus vulgaris, Proteus mirabilis and Shigella flexneri.

**8.** A method for preparing the compound as shown in the following formula Ia,

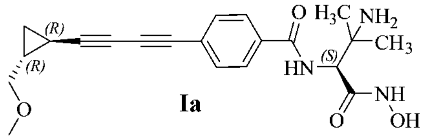

Ia

which comprises one or more of the following steps:

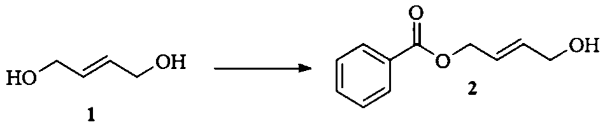

1 2

Step 1

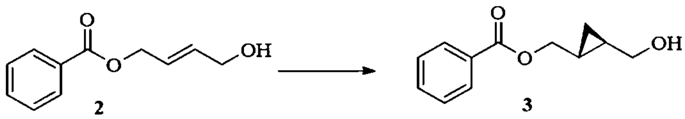

2 3

Step 2

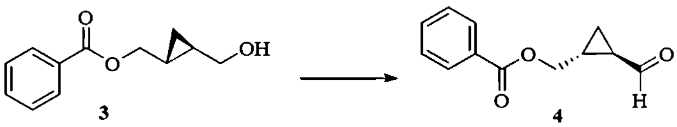

3 4

Step 3

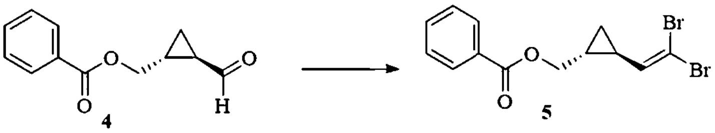

4 5

Step 4

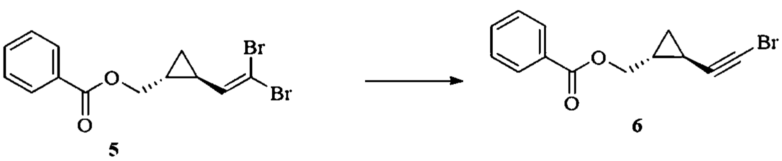

5 6

Step 5

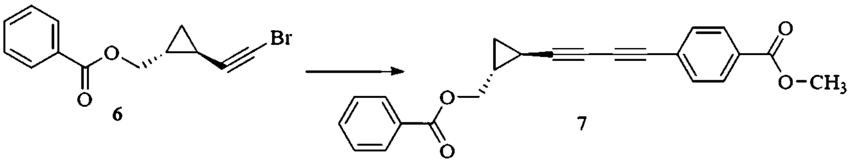

6 7

Step 6

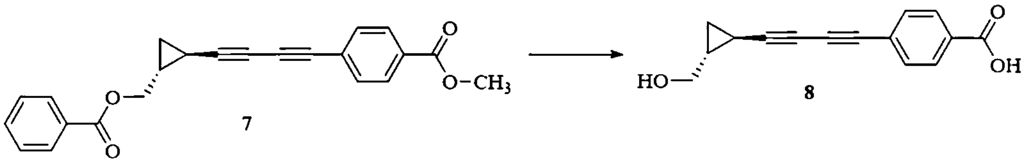

7 8

Step 7

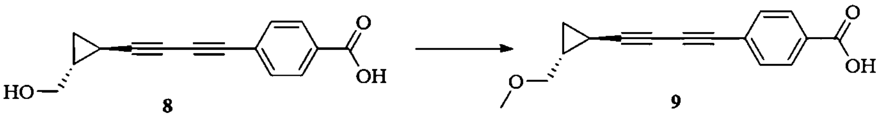

8 9

Step 8

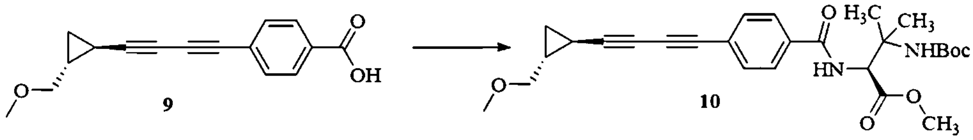

9 10

Step 9

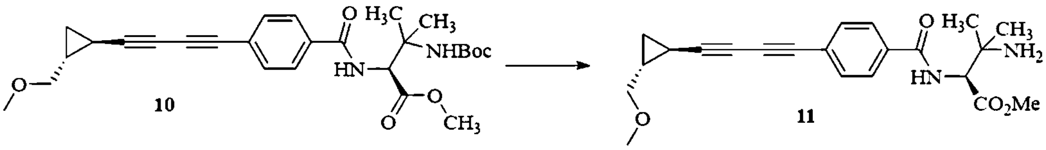

10 11

Step 10

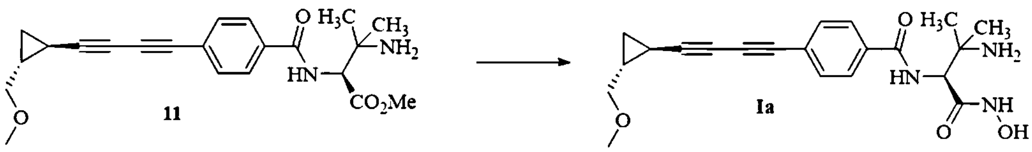

11 Ia

Step 11.

**9.** The preparation method according to claim 8, wherein an asymmetric inducer is employed in Step 2.

**10.** A method for preparing the compound as shown in the following formula Ib, Ic or Id,

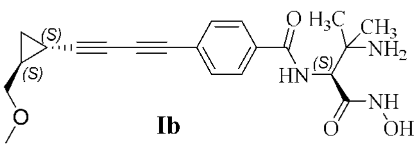

Ib

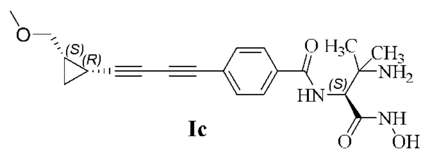

Ic

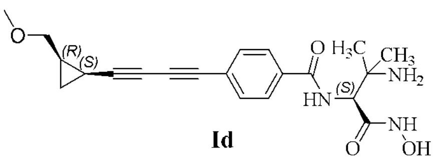

Id

which comprises any one or more of the following steps, wherein Ib is prepared using *trans*-2-butene-1,4-diol as the starting material, and Ic and Id are prepared using *cis*-2-butene-1,4-diol as the starting materials:

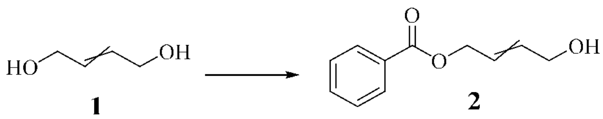

1 2

Step 1'

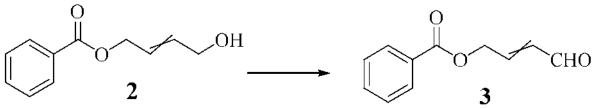

2 3

Step 2'

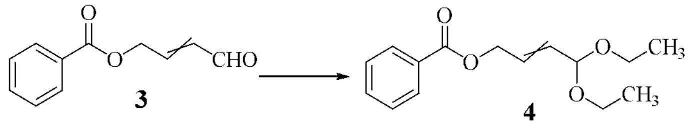

3 4

Step 3'

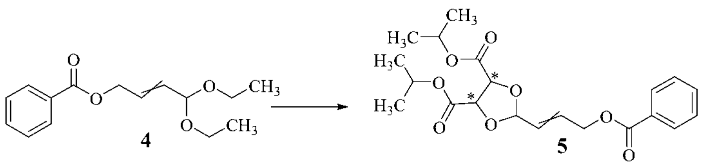

4 5

Step 4'

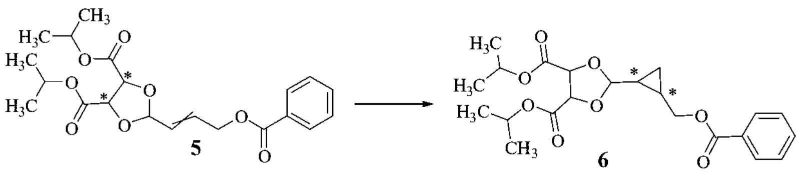

5 6

Step 5'

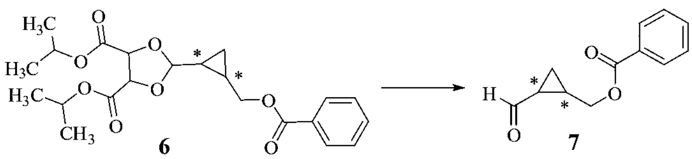

6 7

Step 6'

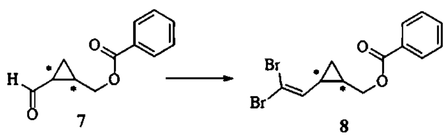

7 8

Step 7'

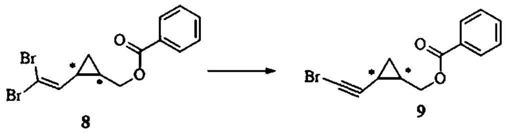

8 9

Step 8'

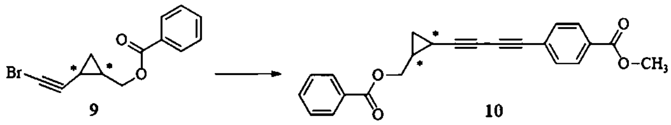

9 10

Step 9'

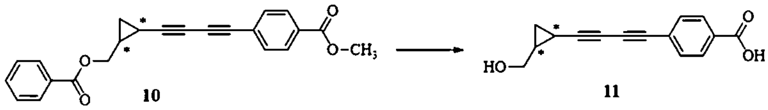

10 11

Step 10'

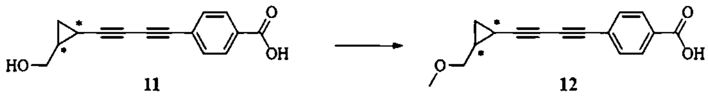

11 12

Step 11'

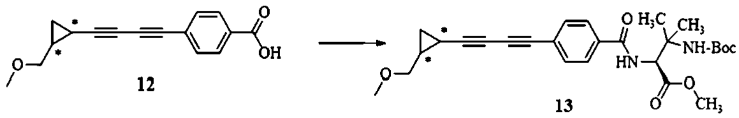

12 13

Step 12'

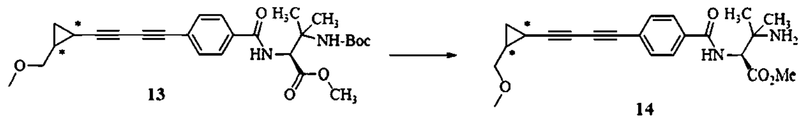

13 14

Step 13'

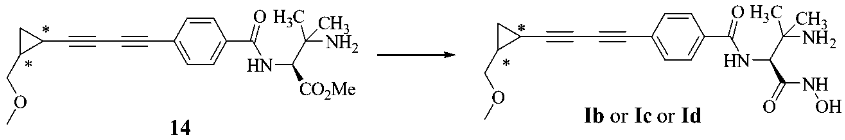

14 Ib or Ic or Id

Step 14' .

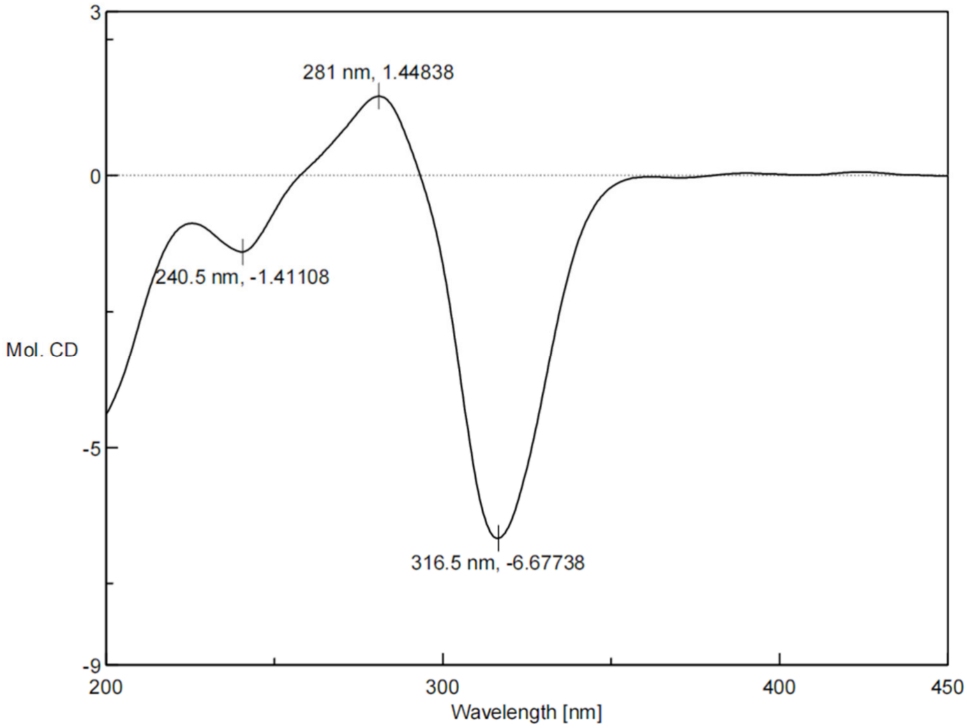
3
0
-5
-9
Mol. CD
281 nm, 1.44838
240.5 nm, -1.41108
316.5 nm, -6.67738
200
300
400
450
Wavelength [nm]

Fig. 1

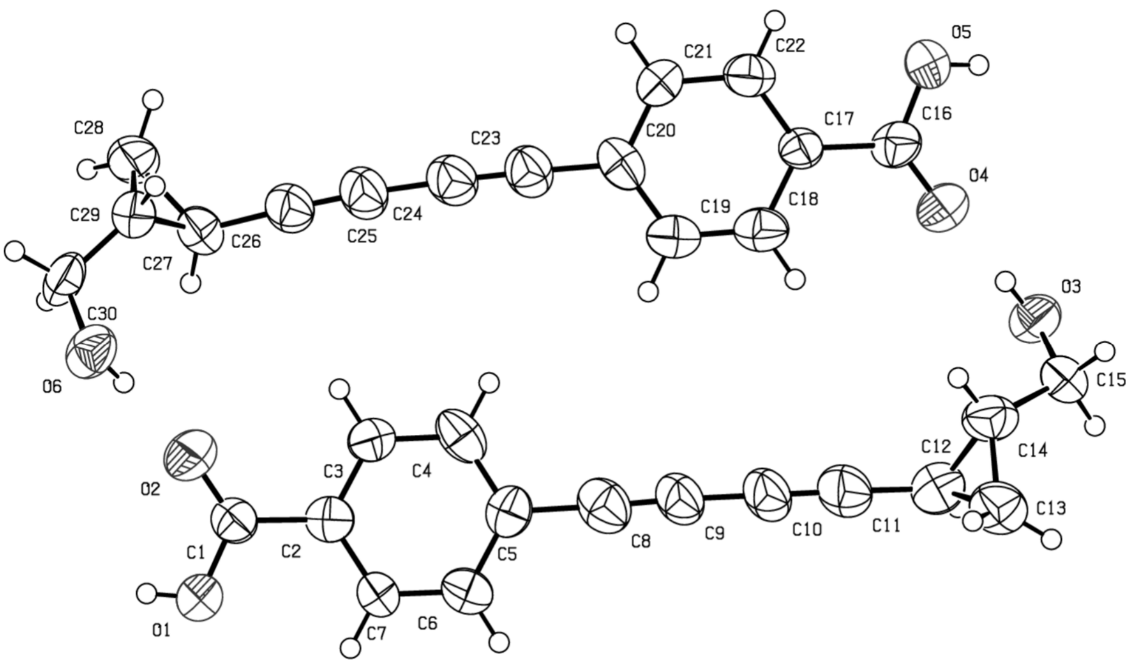

Fig. 2

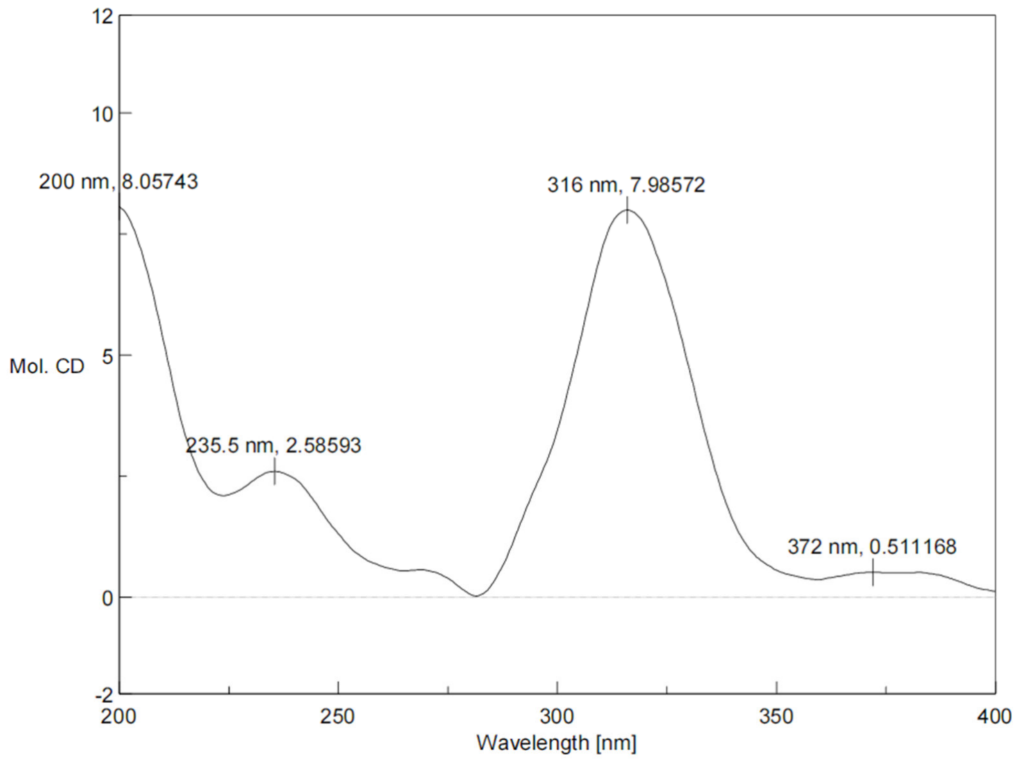
Mol. CD
Wavelength [nm]
200 nm, 8.05743
235.5 nm, 2.58593
316 nm, 7.98572
372 nm, 0.511168
12
10
5
0
-2
200
250
300
350
400

Fig. 3

**INTERNATIONAL SEARCH REPORT**

International application No.
**PCT/CN2024/101528**

**A. CLASSIFICATION OF SUBJECT MATTER**

C07C259/06(2006.01)i; A61K31/166(2006.01)i; A61P31/04(2006.01)i

According to International Patent Classification (IPC) or to both national classification and IPC

**B. FIELDS SEARCHED**

Minimum documentation searched (classification system followed by classification symbols)

IPC: C07C, A61K, A61P

Documentation searched other than minimum documentation to the extent that such documents are included in the fields searched

Electronic data base consulted during the international search (name of data base and, where practicable, search terms used)

CNTXT, VEN, ENTXTC, CNKI, 万方, WANFANG, Web of Science, STN, ACHN-975, 苯甲酰胺, 苯基甲酰胺, 丁基, 二炔, benzamide, butadiyne, butane, diyne

**C. DOCUMENTS CONSIDERED TO BE RELEVANT**

| Category* | Citation of document, with indication, where appropriate, of the relevant passages | Relevant to claim No. |
|---|---|---|
| PX | CN 116730870 A (INSTITUTE OF MEDICINAL BIOTECHNOLOGY, CHINESE ACADEMY OF MEDICAL SCIENCES) 12 September 2023 (2023-09-12) claims 1-10 | 1-10 |
| PX | WO 2024077160 A2 (DUKE UNIVERSITY) 11 April 2024 (2024-04-11) claims 1-71 | 1-10 |
| X | CN 110563611 A (INSTITUTE OF MEDICINAL BIOTECHNOLOGY, CHINESE ACADEMY OF MEDICAL SCIENCES) 13 December 2019 (2019-12-13) claims 1-10, and description, paragraphs [0004]-[0038], [0119]-[0135], and [0170]-[0179] | 1, 5-7 |
| X | CN 103298780 A (ACHAOGEN, INC.) 11 September 2013 (2013-09-11) claims 1-23, and description, paragraphs [0013]-[0025], [0079]-[0083], and [0141]-[0219] | 1, 5-7 |
| X | US 2019169114 A1 (DUKE UNIVERSITY et al.) 06 June 2019 (2019-06-06) claims 1-7, and description, paragraphs [0006]-[0052] and [0208]-[0218] | 1-10 |
| Y | US 2019169114 A1 (DUKE UNIVERSITY et al.) 06 June 2019 (2019-06-06) claims 1-7, and description, paragraphs [0006]-[0052] and [0208]-[0218] | 8-10 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **13 September 2024** | **11 October 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**

International application No.
**PCT/CN2024/101528**

**C. DOCUMENTS CONSIDERED TO BE RELEVANT**

| Category* | Citation of document, with indication, where appropriate, of the relevant passages | Relevant to claim No. |
|---|---|---|
| Y | CN 103298780 A (ACHAOGEN, INC.) 11 September 2013 (2013-09-11)<br>claims 1-23, and description, paragraphs [0013]-[0025], [0079]-[0083], and [0141]-[0219] | 8-10 |
| Y | CHARETTE, A. B. et al. "Diastereo- and Enantioselective Synthesis of 1,2,3-substituted Cyclopropanes with Zinc Carbenoids"<br>*Angen. Chem. Inr. Ed. Engl.,* Vol. 36, No. 10, 15 May 1997 (1997-05-15), pages 1090-1092<br>ISSN: 1521-3773,<br>page 1091, Scheme 2 and page 1092, Experimental Section | 8-9 |
| A | CN 105777464 A (SHANGHAI INSTITUTE OF MATERIA MEDICA, CHINESE ACADEMY OF SCIENCES) 20 July 2016 (2016-07-20)<br>claims 1-10 | 1-10 |

Form PCT/ISA/210 (second sheet) (July 2022)

**INTERNATIONAL SEARCH REPORT**
**Information on patent family members**

International application No.
**PCT/CN2024/101528**

| Patent document cited in search report | | | Publication date (day/month/year) | Patent family member(s) | | | Publication date (day/month/year) |
|---|---|---|---|---|---|---|---|
| CN | 116730870 | A | 12 September 2023 | CN | 116730870 | B | 13 October 2023 |
| WO | 2024077160 | A2 | 11 April 2024 | WO | 2024077160 | A3 | 23 May 2024 |
| CN | 110563611 | A | 13 December 2019 | WO | 2021052353 | A1 | 25 March 2021 |
| | | | | US | 2022033369 | A1 | 03 February 2022 |
| | | | | US | 11827612 | B2 | 28 November 2023 |
| | | | | JP | 2022516195 | A | 24 February 2022 |
| | | | | EP | 4071134 | A1 | 12 October 2022 |
| | | | | EP | 4071134 | A4 | 21 December 2022 |
| | | | | CN | 110563611 | B | 02 February 2021 |
| CN | 103298780 | A | 11 September 2013 | TW | 201249786 | A | 16 December 2012 |
| | | | | JP | 2014501716 | A | 23 January 2014 |
| | | | | IL | 226270 | A0 | 31 July 2013 |
| | | | | AR | 083760 | A1 | 20 March 2013 |
| | | | | MX | 2013005200 | A | 28 June 2013 |
| | | | | KR | 20140023869 | A | 27 February 2014 |
| | | | | AU | 2011367819 | A1 | 02 May 2013 |
| | | | | CA | 2817211 | A1 | 15 November 2012 |
| | | | | EP | 2638006 | A1 | 18 September 2013 |
| | | | | BR | 112013011693 | A2 | 09 August 2016 |
| | | | | EA | 201390626 | A1 | 30 December 2013 |
| | | | | WO | 2012154204 | A1 | 15 November 2012 |
| | | | | SG | 190243 | A1 | 28 June 2013 |
| US | 2019169114 | A1 | 06 June 2019 | ES | 2961564 | T3 | 12 March 2024 |
| | | | | EP | 4286001 | A2 | 06 December 2023 |
| | | | | EP | 4286001 | A3 | 17 July 2024 |
| | | | | EP | 3448375 | A1 | 06 March 2019 |
| | | | | EP | 3448375 | A4 | 11 December 2019 |
| | | | | EP | 3448375 | C0 | 27 September 2023 |
| | | | | US | 2019367446 | A1 | 05 December 2019 |
| | | | | US | 11034649 | B2 | 15 June 2021 |
| | | | | US | 2021206715 | A1 | 08 July 2021 |
| | | | | WO | 2017189586 | A1 | 02 November 2017 |
| | | | | CA | 3022102 | A1 | 02 November 2017 |
| | | | | US | 10550074 | B2 | 04 February 2020 |
| | | | | JP | 2019520317 | A | 18 July 2019 |
| | | | | JP | 7012289 | B2 | 14 February 2022 |
| | | | | AU | 2017257755 | A1 | 22 November 2018 |
| | | | | AU | 2017257755 | B2 | 06 May 2021 |
| CN | 105777464 | A | 20 July 2016 | CN | 105777464 | B | 29 September 2020 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- CN 10329878 A
- CN 101765585 A
- CN 103298780 A
- CN 110563611 A

### Non-patent literature cited in the description

- *CHEMICAL ABSTRACTS*, 110-71-4